# EUROPEAN PATENT SPECIFICATION

(11) **EP 4 431 071 B1**
(45) Date of publication and mention of the grant of the patent: **25.03.2026**
(21) Application number: 23214625.8
(22) Date of filing: 06.12.2023
(51) Int. Cl.: A61F 13/494, A61F 13/496, A61F 13/15, A61F 13/505

(54) **METHOD AND APPARATUS FOR THE MANUFACTURE OF ABSORBENT ARTICLE WITH TRANSVERSAL BARRIER**
VERFAHREN UND VORRICHTUNG ZUR HERSTELLUNG EINES SAUGFÄHIGEN ARTIKELS MIT QUERBARRIERE
PROCÉDÉ ET APPAREIL POUR LA FABRICATION D'UN ARTICLE ABSORBANT À BARRIÈRE TRANSVERSALE

(30) Priority: 13.03.2023 EP 23161594; 12.10.2023 EP 23203360
(43) Date of publication of application: 18.09.2024
(73) Proprietor: Ontex BV, 9255 Buggenhout (BE); Ontex Group NV, 9320 Erembodegem (BE)
(72) Inventor: HORNE, Jacob Philip, D-56727 Mayen (DE); FRANSEN, Markus, 56269 Dierdorf (DE); PSCHYKLENK, Lukas, 56727 Müllenbach (DE); BUYSSE, Michiel, 9968 Oosteeklo (BE); IFFLAND, Dirk, D-45529 Hattingen (DE)
(74) Representative: Saurat, Thibault

(56) References cited:
- EP-A1- 2 612 637
- US-B1- 6 221 460
- US-B2- 7 204 830
- US-B2- 9 789 008

## Description

### TECHNICAL FIELD

The present disclosure is directed to an absorbent article for personal hygiene, typically in the form of pants (such as for babies, youths or adults) and generally of the disposable kind as well as the method for the manufacture of such absorbent articles and the apparatus to carry out such method.

### BACKGROUND

Absorbent articles for personal hygiene are designed to absorb and contain bodily exudates, such as a large quantity of urine and/or stool. Non-limiting examples of disposable absorbent articles include diapers, pants, training pants, pad, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp (also referred to as fluff pulp and/or cellulose fibers) with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP3451989 B1) directed to limiting the risk of leakage by providing transversal barriers at the front and/or back of a diaper. However whilst such executions may be effective on diaper constructs they are less effective and or complex/costly to incorporate into a pant concept.

An attempt to overcome such shortcomings has been made in for example WO2021/170027 A1 wherein a nonwoven layer of the front and/or back belt is folded a plurality of times to form a waist guard. Although this provides for an effective way to introduce a transversal barrier in a pant construct, there are several drawbacks that have been identified: firstly, the nonwoven that is generally hydrophobic may be sufficient to capture solids but is less effective to capture larger amounts of liquid exudates (particularly under pressure such as when a baby is moving and/or application of sudden pressure such as sitting/dropping on the floor on its buttocks); secondly it limits the positioning thereof to a closer position at the waist edge which is less effective in providing a barrier since it is positioned further away from the core and to position it closer to the core and away from the waist edge would require a significant increase in length of the nonwoven which adds significant waste and cost; thirdly applying the multiple folds at different positions of the same component and especially further elasticizing the region when/if desired may add significant process complexity particularly at high speeds. Apparatuses for conveying, folding and joining are also known from EP2612637 A1 and US9789008 B2. Folding absorbent inserts is known from US6221460 B1 and from US7204830 B2.

A need therefore exists for improved absorbent articles having leakage protection particularly for substantially liquid exudates such as urine and/or liquid stool yet in in a cost-effective manner and with more limited additional waste as well as having an efficient method and apparatus for the manufacture of such absorbent articles.

### SUMMARY

The disclosure relates to an absorbent article for personal hygiene, preferably a disposable pant, comprising: a front panel preferably substantially transversely extending; a back panel preferably substantially transversely extending; and an absorbent insert, preferably substantially longitudinally extending, joined to each of said front and back panels, said absorbent insert comprising front and back transversal edges and left and right longitudinal edges connecting the front and back transversal edges to form a perimeter thereof and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween). The front and back panels are joined together to define a pair of side seams, and at least a portion of said absorbent insert proximal to the front and/or back transversal edges thereof comprises a transversal waist barrier comprising a substantially liquid impermeable film layer.

The present disclosure pertains to a method for the manufacture of an absorbent article (1) comprising the steps of: providing a front panel ; providing a back panel ; providing an absorbent insert comprising front and back transversal edges and left and right longitudinal edges connecting the front and back transversal edges to form a perimeter thereof; joining said absorbent insert to said front and/or back panels followed by folding a portion of said absorbent insert proximal to the front and/or back transversal edges thereof onto itself, preferably in the shape of a substantially U-fold, to form transversal waist barrier(s); and joining said front and back panels together along a pair of oppositely disposed side seams. In other terms, the method comprises a plurality of steps with the step of joining the absorbent insert to said front and/or back panels being prior to the step of folding a portion of said absorbent insert proximal to the front and/or back transversal edges thereof onto itself. In other words, said folding step, where a portion of said absorbent insert proximal to the front and/or back transversal edges thereof is folded onto itself, occurs after said joining step where said absorbent insert is joined to the front and/or back panels. Said folding step can occur directly after, meaning sequentially after, or subsequentially, the joining step, or the present disclosure encompasses embodiments with additional steps occurring between said joining step and said folding step, for example a step where adhesive is intermittently applied onto a portion of the absorbent insert, a step where said absorbent insert is conveyed downstream from said joining step to said folding step. The present disclosure also encompasses embodiments where the joining step and the folding step are partially concurrent, for example, the absorbent insert is started to be folded even though the absorbent insert is not entirely joined to the front and/or back panels. Preferably, said folding step is carried out by static folding where a static element, *i.e.* a stator, such as a stationary rail or a stationary rod or a stationary folding blade, said static element deflects a portion of said absorbent insert proximal to the front and/or back transversal edges thereof onto itself. In other words, the folding is step is preferably done by passive folding meaning that the folding is done with a moving element, i.e. the absorbent insert, being folded by an unmoving, or immobile or fixed element, e.g. a rail or a rod or a plate or a blade. Such folding is more cost efficient and more reproducible. The present disclosure encompasses embodiments where the folding step is carried out by dynamic folding, where a mobile element, e.g. an actuated folding blade or folding rail, moves or is actuated to fold a portion of said absorbent insert proximal to the front and/or back transversal edges thereof onto itself. In other words, the method preferably comprises a step such as statically folding a portion of said absorbent insert proximal to the front and/or back transversal edges thereof onto itself, preferably in the shape of a substantially U-fold, to form transversal waist barrier(s).

According to an embodiment, the method further comprising the steps of folding an outer nonwoven layer of the front and/or back panels over the folded absorbent insert and joining said folded outer nonwoven layer directly or indirectly to the folded absorbent insert. Preferably, these said steps occur later, or after, said folding step, meaning after a portion of said absorbent insert proximal to the front and/or back transversal edges thereof is folded onto itself and prior to the step where said front and back panels are joined together along a pair of oppositely disposed side seams.

According to an embodiment, the method further comprising the step of arranging an elastic material over the folded portion of the folded absorbent insert prior to step of folding an outer nonwoven layer of the front and/or back panels over the folded absorbent insert. Preferably the elastic material is arranged such that said elastic material is sandwiched between the absorbent insert and the outer nonwoven layer. More preferably, the absorbent insert comprises a backsheet and eventually an outer cover on the garment facing surface of the absorbent core, the elastic material being sandwiched between the backsheet and the outer nonwoven layer or the elastic material being sandwiched between the outer cover and the outer nonwoven layer or the elastic material being sandwiched between the backsheet and outer cover and the outer nonwoven layer e.g. in cases where the outer cover is transversally shorter than the backsheet. In other words, the elastic material is directly or indirectly joined to the backsheet, preferably by adhesive, and sandwiched between the backsheet and/or outer cover and the outer nonwoven layer. Preferably, this said step occurs following, or after, said folding step, meaning after a portion of said absorbent insert proximal to the front and/or back transversal edges thereof is folded onto itself.

According to an embodiment, the method further comprising the step of intermittently applying adhesive, preferably in the machine direction, onto said portion of the absorbent insert proximal to the front and/or back transversal edges.

According to an embodiment, the method further comprising the step of arranging an elastic material over the folded portion of the folded absorbent insert prior to step of folding an outer nonwoven layer of the front and/or back panels over the folded absorbent insert.

According to an embodiment, the method further comprising the step of intermittently applying adhesive onto said portion of the absorbent insert proximal to the front and/or back transversal edges.

According to an embodiment, the method further comprising the step of applying adhesive onto said portion of the absorbent insert proximal to the front and/or back transversal edges in an adhesive pattern, said adhesive pattern comprising at least two areas of adhesive arranged at the transversal extremities of the front and/or back transversal edges, a further area of adhesive extending transversally between said two areas of adhesive, said further area being at a longitudinal distance from the transversal edge, and an adhesive-free area arranged between the transversal edge of the absorbent insert and said further area.

According to an embodiment, the method further comprising the step of compressing the folded portion of the folded absorbent insert, preferably at least the transversal waist barrier(s), once formed, said step being prior to the steps of folding an outer nonwoven layer of the front and/or back panels over the folded absorbent insert and joining said folded outer nonwoven layer directly or indirectly to the folded absorbent insert. Preferably, this step being prior to the step of arranging an elastic material over the folded portion of the folded absorbent insert prior to step of folding an outer nonwoven layer of the front and/or back panels over the folded absorbent insert.

According to an embodiment, the method further comprising the step of strand coating the elastic material prior to the step of arranging said elastic material over the folded portion of the folded absorbent insert.

According to an embodiment, the method further comprising the step of compressing the folded portion of the folded absorbent insert once the outer nonwoven layer has been joined directly or indirectly to the folded absorbent insert.

According to an embodiment, the method further comprising the step of mechanically bonding the folded transversal edge of the absorbent insert once the transversal waist barrier(s) has been formed.

In another aspect, the disclosure relates to an apparatus for the manufacture of an absorbent article according to a method as described herein wherein the apparatus comprises:
- a conveying device, preferably a conveyor belt, for conveying said front and/or back panels and for joining said absorbent insert to said front and/or back panels; in other words, the apparatus comprises a conveying device for conveying front and/or back panels and an absorbent insert joined to said front and/or back panels.
- a folding unit for folding a portion of said absorbent insert proximal to the front and/or back transversal edges thereof onto itself, preferably in the shape of a substantially U-fold, to form transversal waist barrier(s), said absorbent insert being joined to the front and/or back panels;
- said absorbent insert;
- a bonding unit for joining said front and back panels together along a pair of oppositely disposed side seams.

According to the present disclosure, the folding unit is arranged downstream of the conveying device conveying the front and/or back panels and joining the absorbent insert to said front and/or back panels, and upstream of bonding unit joining said front and back panels together along a pair of oppositely disposed side seams.

According to an embodiment, the apparatus further comprises a transfer device for transferring absorbent inserts assembled beforehand, said transfer device, or transfer unit, comprising a plurality of transfer heads arranged on a support wheel with both the support wheel and the transfer heads being configured to respectively rotate around a respective axis. According to this embodiment, said conveying device is arranged downstream of the transfer device so that the conveying device can collect or receive or pick up the absorbent insert or in other words, the transfer device can deposit each absorbent insert onto the conveying device conveying the front and/or back panels. **The** transfer device is an apparatus that is configured to turn and repitch the discrete absorbent inserts assembled beforehand, turn the discrete absorbent inserts, or merely transfer the discrete absorbent inserts between a pick-up location and a drop-off location.

According to an embodiment, the conveying device extends in the vertical direction, said apparatus extending in a machine direction, a cross-direction and a vertical direction.

According to an embodiment, the present disclosure relates to an apparatus for the manufacture of an absorbent article according to a method as described herein wherein the apparatus comprises:
- a conveying device, preferably a conveyor belt, for conveying an absorbent insert;
- a folding unit for folding a portion of said absorbent insert proximal to the front and/or back transversal edges thereof onto itself, preferably in the shape of a substantially U-fold, to form transversal waist barrier(s);
- a rotating drum and/or a second conveying device, preferably a conveyor belt, for joining said insert to said front and back panels;
- a bonding unit for joining the joining said front and back panels together along a pair of oppositely disposed side seams.

According to an embodiment, the folding unit comprises, preferably consists of, a static element, preferably in the form of a rail or a rod or a folding blade. **The** static element is elongated and extends in the machine direction and/or cross direction and/or vertical direction. **The** static element can either extend linearly, meaning in a substantially straight line, or curvy, meaning in a curved line. **The** static element can extend either in one direction, such as in the machine direction or in the cross direction or in two directions such as diagonally in the machine and cross direction or in three directions such as diagonally in the machine, cross and vertical directions. **The** static element can comprise a curvature, said curvature spanning over an angle from 0° to at least 180°, preferably at least 200°, more preferably at least 250°, for example 275°.

According to an embodiment, the folding unit further comprises a pressing blade arranged at a distance from the folding blade with respect to the cross direction and/or vertical direction and arranged to press on absorbent insert at a portion located between the portion of the absorbent insert proximal to the front and/or back transversal edges and the central portion of the absorbent insert.

According to an embodiment, the pressing blade is shorter in length, with respect to the machine direction.

According to an embodiment, the pressing blade comprise a sloped portion at the upstream portion of the pressing blade with respect to the machine direction, said sloped portion extending diagonally downward while extending downstream with respect to the machine direction.

According to an embodiment, the apparatus comprises an adhesive dispensing device arranged upstream or downstream of the folding unit with respect to the machine direction, said device being configured to apply adhesive intermittently.

According to an embodiment, the adhesive is applied in swirls or in a uniform application.

According to an embodiment, a pressure roller is arranged to press the folded transversal edge of the absorbent insert, said pressure roller being arranged downstream of the folding unit and upstream an elastic depositing unit or station, where a second elastic material, e.g. one or more elastic strands, is deposited onto the backsheet or the outer cover of the absorbent insert at the region that has been folded over

According to an embodiment, the folding unit comprises a support device to carry the static element and/or pressing blade said support device comprising means to move the static element and/or pressing blade or comprising means to remove the static element and/or pressing blade, preferably in the cross and/or vertical directions (CD,Z).

According to an embodiment, the folding unit comprises an adjustable mechanism to adjust the distance between the pressing blade and the static element with respect to the cross direction and/or vertical direction.

According to an embodiment, the folding unit comprises two static elements, preferably each with a pressing blade, said static elements being arranged at each end of the conveying device with respect to the cross direction, the static elements being substantially identical in dimensions and shape.

These embodiments can be taken alone or in combination.

### BRIEF DESCRIPTION OF THE FIGURES

**FIG. 1** is a top planar view of an absorbent article according to an embodiment of the present disclosure.
**FIG. 2** is a top planar view of an absorbent article according to an embodiment of the present disclosure.
**FIG. 3** is a partial cross-section of an absorbent article according to an embodiment of the present disclosure.
**FIG. 4** is a top planar view of an absorbent article according to an embodiment of the present disclosure.
**FIG. 5** is a side view, or profile view, of the apparatus for the manufacture of absorbent articles according to an embodiment of the present disclosure.
**FIG. 6** is a view in perspective of an isolated component, the folding blade, of the apparatus of FIG. 5.
**FIG. 7** is a view in perspective of an isolated component, the folding unit, of the apparatus of FIG. 5.
**FIG. 8** is a cross section of the folding blade at the downstream portion with respect to the machine direction MD.
**FIG. 9** is a top planar view of an absorbent article according to an embodiment of the present disclosure.
**FIG. 10A** is a top planar view of an absorbent article according to an embodiment of the present disclosure prior to the folding of the portion of said absorbent insert proximal to the back transversal edge thereof.
**FIG. 10B** is a top planar view of an absorbent article according to FIG. 10A after folding of the portion of said absorbent insert proximal to the back transversal edge thereof.
**FIG. 11** is a top planar view of an absorbent article according to an embodiment of the present disclosure.
**FIG. 12** is a top planar view of an absorbent article according to an embodiment of the present disclosure.
**FIG. 13** is a top planar view of a first conveying device conveying front and back panels and absorbent inserts according to an embodiment of the present disclosure illustrating different steps in the method.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garmentside" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

"Front" and "rear or back" of the absorbent article as used herein typically refer for the "front" end of the article is that which is most proximal to the front of the subject when worn, the "rear or back" end of the article is that which is most proximal to the rear or back of the subject when worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The absorbent articles of the disclosure will be further illustrated in the below description and in the Figures, though all embodiments described herein may equally be applied onto absorbent articles in the form of pants (or even in the form of feminine hygiene articles such as menstrual pants and/or slips/panties). Nothing in this description should be however considered limiting the scope of the claims unless explicitly indicated otherwise. Unless indicated otherwise, the description refers to the dry article, i.e. before use and conditioned at least 24 hours at 21° C.+/-2° C. and 50+/-20% Relative Humidity (RH).

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The terms "joined" or "associated" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further include embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

As used herein, the terms "machine direction", "cross direction" and "vertical direction" are with respect to the apparatus suitable for forming absorbent articles with transversal barrier(s) in an assembled state or in a final installation state, meaning once normally functioning. The term "in the direction" as used herein means along that direction, e.g. in the machine direction means along the machine direction.

The values indicated herein are measured according to the methods indicated herein below, unless specified otherwise. All measurements are performed at 21±2° C. and 50±20% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

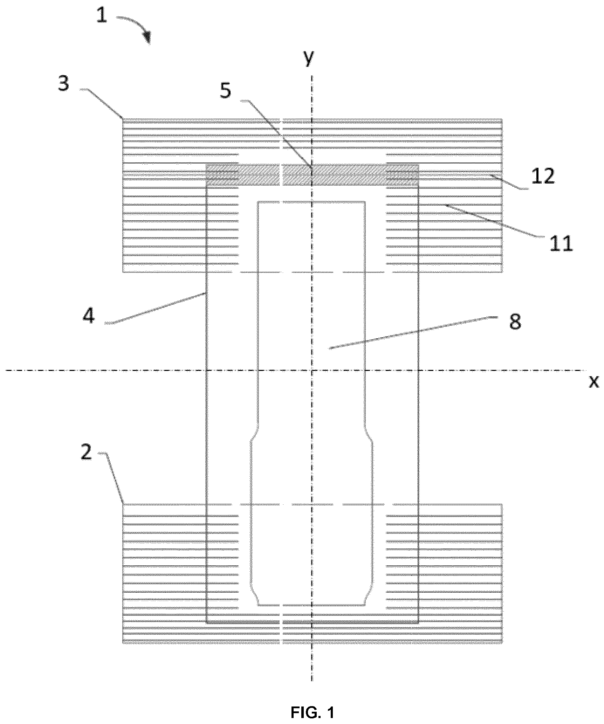

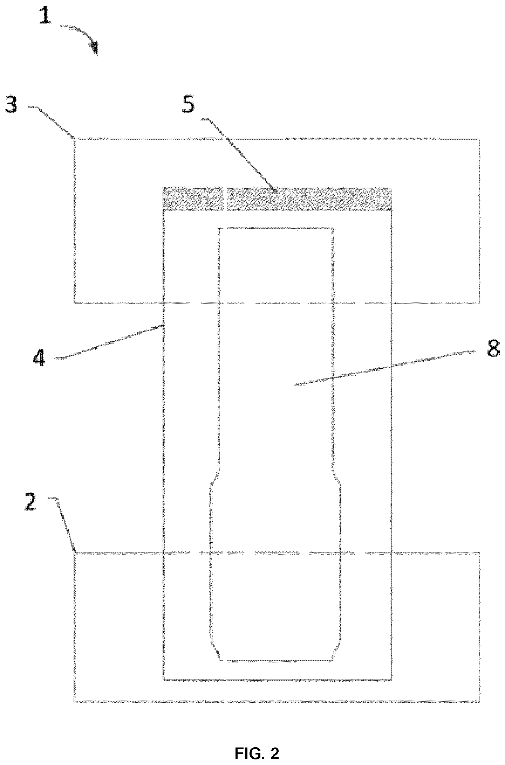

As exemplified in FIG. 1 to FIG. 3, absorbent articles herein are for personal hygiene, preferably in the form of a pant, the absorbent article comprising: a substantially transversely extending front panel (2); a substantially transversely extending back panel (3); and a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof (generally wherein the left and right longitudinal edges extend substantially parallel to the longitudinal axis y and are oppositely disposed such that said longitudinal axis y extends therebetween, and the front and back transversal edges extend substantially parallel to the transversal axis x, and typically substantially perpendicular to the longitudinal axis y, and are oppositely disposed such that said transverse axis x extends therebetween); wherein the front and back panels (2, 3) are joined together to define a pair of side seams, and wherein at least a portion of said absorbent insert (4) proximal to the front and/or back transversal edges thereof comprises a transversal waist barrier(s) (5) comprising a substantially liquid impermeable film layer, preferably wherein said transversal waist barrier (5) is an up-standing barrier generally arranged to block substantially liquid exudates from flowing therethrough. Advantageously, this arrangement allows for an absorbent article with superior leakage prevention particularly for substantially liquid exudates such as urine and/or liquid stool.

Generally, the substantially liquid impermeable film layer is an integral component of at least one element or portion of the absorbent article such as a backsheet (7) thereof typically such that no additional and/or separate layer, element and/or material is incorporated to form the transversal waist barrier(s) (5). Advantageously this allows to reduce waste and/or cost associated with incorporating separate elements to create the transversal waist barrier(s).

The backsheet (7) is generally that portion of the absorbent article positioned adjacent the garment-facing surface of the absorbent core (8) and which prevents the exudates absorbed and contained therein from soiling articles such as bedsheets and undergarments. The backsheet (7) is typically impermeable to liquids (e.g. urine). The backsheet (7) may for example be or comprise a thin plastic film such as a thermoplastic film having a thickness of about 0.012 mm to about 0.051 mm. Preferably the basis weight of the backsheet (7) is less than or equal to 16 g/m², more preferably from 10 g/m² to 14 g/m². Exemplary backsheet films include those manufactured by Tredegar Corporation, based in Richmond, Va., and sold under the trade name CPC2 film. Other suitable backsheet materials may include breathable materials which permit vapors to escape from the absorbent article while still preventing exudates from passing through the backsheet (7). Exemplary breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, Va., and sold under the designation EXAIRE, and monolithic films such as manufactured by Clopay Corporation, Cincinnati, Ohio under the name HYTREL blend P18-3097. Some breathable composite materials are described in greater detail in PCT Application No. WO 95/16746 published on Jun. 22, 1995 in the name of E. I. DuPont; U.S. Pat. No. 5,938,648 to LaVon et al., U.S. Pat. No. 4,681,793 to Linman et al., U.S. Pat. No. 5,865,823 to Curro; and U.S. Pat. No. 5,571,096 to Dobrin et al, U.S. Pat. No. 6,946,585B2 to London Brown.

In a preferred embodiment the backsheet (7) is breathable. Breathable backsheet herein typically means that it comprises micro-openings sized to allow at least some water vapour to permeate through the backsheet that typically comprises a film such as a polyethylene (PE) film.

Preferably a backsheet (7) is breathable when the water vapour transmission rate (WVTR) of the backsheet is at least 500 grams/m2 - 24 hours, preferably at least 1,000 grams/m2 - 24 hours, even more preferably from 1,200 grams/m2 - 24 hours to 2,500 grams/m2 - 24 hours, even more preferably from 1,500 grams/m2 - 24 hours to 2,100 grams/m2 - 24 hours, as measured according to ASTM D6701-21.

The backsheet (7) may be joined to the topsheet (6), the absorbent core (8) or any other element of the absorbent article by any attachment means known in the art. Suitable attachment means are described above with respect to means for joining the topsheet to other elements of the article. For example, the attachment means may include a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minn. and marketed as HL-1620 and HL 1358-XZP. Alternatively, the attachment means may comprise heat bonds, pressure bonds, ultrasonic bonds, dynamic mechanical bonds, or any other suitable attachment means or combinations of these attachment means as are known in the art.

The absorbent core (8) of the absorbent article may comprise one or more core layers. As explained, the absorbent article may comprise an acquisition distribution system, which will typically consist of one or more layers. Most typically, the layers are arranged above the core layer. Hence, a number of layers can be arranged between the topsheet and the backsheet. The skilled person will usually have no difficulty in distinguishing between these layers. In case of doubt, a core layer can be identified as being a layer which is generally less permeable than a layer forming part of the acquisition-/distribution-system.

Permeability generally refers to the quality of a porous material that causes it to a lower liquid or gases to pass through it. Hence, the layers of the acquisition distribution system should generally be more permeable than the layers of the core system as these layers are meant to distribute liquid to the absorbent core, where the liquid is ultimately stored.

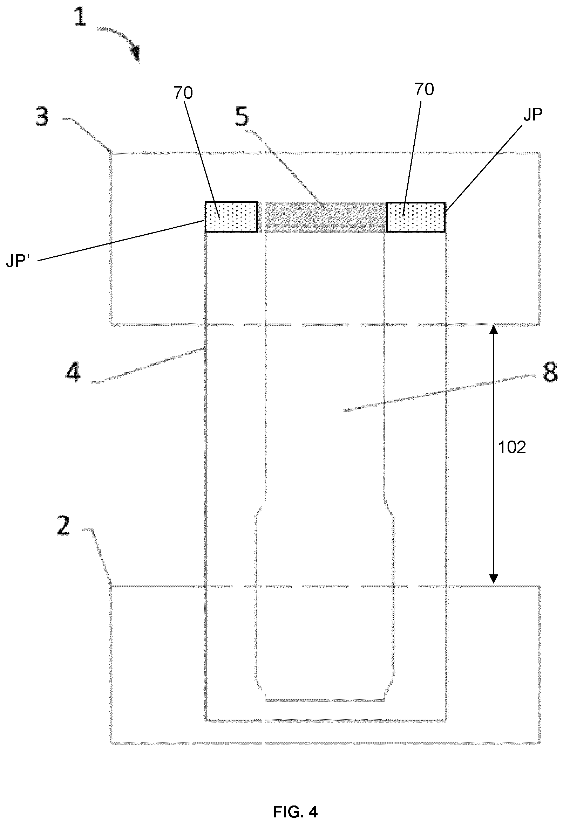

In an embodiment, as exemplified in Fig. 4, the transversal waist barrier(s) (5) overlap at least a portion of an absorbent core (8) of the absorbent insert (4) typically such that the exudates collected and/or retained by the transversal waist barrier(s) (5) are substantially dehydrated and/or absorbed by the neighbouring absorbent core (8) surface(s). Advantageously this allows for reduced leakage risks of highly liquid exudates like urine or liquid stool such as diarrhoea (or other liquid stool that is generally and commonly observed e.g. with new-born babies).

Preferably the front and back panels (2, 3) are separate and connected to each other by the absorbent insert (4) acting as a bridge element to form a substantially H-shaped pant. Advantageously this allows to omit cutting/shaping of panels in a crotch region of the article thus reducing waste and/or limiting the amount of panel material in view of its absence in the crotch region also helping to reduce waste and cost.

The absorbent insert (4) may be joined to the front and back panels (2, 3) by adhesive and/or mechanical bonding such as ultrasonic bonding, pressure bonding, and/or thermal bonding and the like. In a preferred embodiment the absorbent insert (4) is joined to the front and back panels (2, 3) by a discontinuous pattern of adhesive. Advantageously this may help to retain softness and pliability of the laminated structures and reduce stiffness when worn.

In an embodiment, the substantially liquid impermeable film layer comprises, preferably consists of, a backsheet (7) of the absorbent insert (4) and preferably comprises a material comprising, or consisting of, a polyester, more preferably said material being selected from the group consisting of polyethylene, polylactic acid, and polypropylene. Advantageously such transversal waist barrier(s) are not only impermeable to a much higher degree than hydrophobic nonwovens but further are formed by using a component already present in the absorbent article and hence does not require adding any further materials that would impact waste and/or cost.

Preferably, the at least portion of said absorbent insert (4) proximal to the front and/or back transversal edges thereof comprises one or more folds forming the transversal waist barrier(s) (5), preferably wherein said fold is substantially U-shaped. Advantageously such fold of the absorbent insert allows to form a liquid impermeable barrier to exudates, and particularly the U-shape (vs other shapes) allows to form a maximised pocket for exudate collection with the minimum use of material.

Preferably, the folded portion of said absorbent insert (4) is joined to the body-facing surface of the topsheet (6) at a pair of oppositely disposed extremities of the absorbent insert (4) along a portion of the left and right longitudinal edges thereof, generally wherein said oppositely disposed extremities are connected by a fold of said folded portion of said absorbent insert (4), such that a pocket is formed that acts as a barrier to exudates in both the longitudinal direction (i.e. substantially parallel to the longitudinal axis y) and the transverse direction (i.e. substantially parallel to the transverse axis x). The said extremities of the absorbent insert (4) may be joined by adhesive and/or mechanical bonding such as ultrasonic bonding, thermal bonding, and/or pressure bonding and the like.

In an embodiment, the absorbent insert (4) comprises a substantially liquid permeable topsheet (6), a substantially liquid impermeable backsheet (7), and an absorbent core (8) sandwiched therebetween, preferably wherein the absorbent core (8) is positioned inboard of the perimeter of said absorbent insert (4) when viewed in a planar direction (as generally depicted and illustrated in the figures herein and that typically corresponds to a plane formed by the x and y axis described and illustrated herein) such that the one or more folds are substantially free of absorbent material. Advantageously, a fold that is substantially free of absorbent material allows for a more flexible fold that can allow the barrier to better stand upright upon application of tension and yet limit piercing of the topsheet and/or backsheet that could arise if exerting a folding force in an area of the absorbent insert comprising SAP particles.

In an embodiment, the font and/or back panels (2, 3) are elastic and comprise an inner nonwoven layer (9), an outer nonwoven layer (10), and an elastic material (11) sandwiched therebetween, wherein the elastic material is selected from the group consisting of an elastic film and a plurality of elastic strands. Preferably, the outer nonwoven layer (10) is wider than the inner nonwoven layer (9) (generally in a direction substantially parallel to the longitudinal axis y) and is folded over said inner nonwoven layer (9) such that it overlaps the portion of said absorbent insert (4) proximal to the front and/or back transversal edges, preferably such that said outer nonwoven layer (10) overlaps both a portion of the backsheet (7) and a portion of the topsheet (6) that remains exposed from said one or more transversal waist barrier(s) (5) such to form a flange (F_{L}). Advantageously this allows to cover the backsheet with nonwoven so that the backsheet does not come into contact with the skin of a wearer which could otherwise result in skin irritations and/or less comfort.

The inner and outer nonwoven layers (9, 10) may be the same or different meaning that they may have the same or different properties selected from basis weight (in gsm) and composition. In an embodiment, both the inner and outer nonwoven layers (9, 10) each comprise a spunbond nonwoven, typically comprising monocomponent fibers of polypropylene, and each have a basis weight of from 10 g/m² (gsm) to 30 g/m² (gsm), preferably from 12 g/m² (gsm) to 25 g/m². The outer nonwoven layer (10) may have a basis weight that is higher than the inner nonwoven layer (9). Advantageously, although it is customary to have the higher basis weight nonwoven on the inner nonwoven wearer skin facing side, in barrier embodiments herein (especially when the outer nonwoven layer is folded and/or extends over the absorbent insert; and/or when the transversal barrier is elasticised) such arrangement provides for added mechanical integrity desirable for resistance to tear on elastification and/or added softness for the wearer on the front and/or back waist regions up to the barrier location.

Preferably, and as exemplified in FIG. 1, the elastic material (11), such as the plurality of elastic strands, is/are deactivated in an area of overlap of the absorbent insert (4) when viewed in a planar direction. The deactivation is preferably achieved by cutting of the elastic strands such as to render the deactivated region of the panel(s) (2, 3) substantially inelastic and regions neighbouring said deactivated region and comprising said elastic strands being elastic. Typically the absorbent core (8) of the absorbent insert (4) is positioned within the deactivated region such that it substantially does not overlap the elastic strands (11). Advantageously this allows for improved core integrity as risks of core collapse due to excessive tension from the stretched elastics when the article is worn is limited.

Preferably more than 55%, preferably more than 60%, even more preferably from 65% to 95%, of the total surface area (generally when viewed in a planar direction) of the transversal waist barrier(s) (5) is positioned to overlap the deactivated region of the panel(s) (2, 3). Advantageously this allows for improved fit of the barrier when the article is worn by a subject.

In addition or alternatively, more than 50%, preferably more than 60%, even more preferably from 65% to 95%, of a total transversal length (generally substantially parallel to the transversal axis x) of the transversal waist barrier(s) (5) overlaps the deactivated region of the panel(s) (2, 3). Especially when the transversal waist barrier(s) is elastic, this arrangement may advantageously not only improve core integrity but further avoid excessive tension build-up.

In an embodiment, the transversal waist barrier(s) (5) is elastic, preferably comprising an elastic material selected from the group consisting of an elastic film and one or more elastic strands. Advantageously this allows for application of tension that permits the barrier to stand upright in close contact to the skin when the article is worn and under stretch.

The elastic strands in any of the embodiments herein may be strand-coated with adhesive prior to joining to one or more layers such as the inner and outer nonwoven layers (9, 10) to form elastic panels (2, 3) and/or the backsheet (7), outer cover (7'), and/or outer nonwoven layer (10) to form the elastic transversal waist barrier(s) (5). Advantageously this allows to join the one or more layers together by the said strand-coated elastic strands and by doing so limit the amount of adhesive used with resulting improved softness as well as reduced waste. Alternatively, or additionally, adhesive in the form of a plurality of stripes is applied such as by slot coating.

In a preferred embodiment, the one or more elastic strands of the transversal waist barrier(s) (5) comprise at least one flat elastic (such as elastics having a cross-section with an aspect ratio of longest to smallest dimension of greater than 1, preferably greater than 1.5, even more preferably greater than 2), preferably in combination with at least one or at least two round elastics (such as elastics having a cross-section with an aspect ratio of longest to smallest dimension of about 1). Although flat elastics are generally more expensive, they are advantageous in providing better gasketing effects and comfort on the wearer's skin, by combining flat and normal/round elastics one can achieve the desired gasketing and comfort whist limiting the cost.

When one or more, preferably only one, flat elastic is used, it is preferably positioned adjacent to an apex of the transversal waist barrier(s) (5) that may be closest to a transversal edge of the absorbent insert (4) and further round elastic(s) positioned further from said apex compared to the flat elastic(s). Advantageously this allows for the flat elastic to be positioned at the closest contact position to the wearer with other elastics being inboard (or further away) therefrom hence contributing to the desired tensile strength yet permitting to lower the overall density/dtex of the flat elastic and hence also cost, with limited impact on performance.

When elastic strands are used, they may have a dtex in the range of from 350 to 1100, preferably from 400 to 1000, even more preferably from 450 to 900. When elastic strands are used in all of the front and/or back panels (2, 3) and the transversal waist barrier(s) (5), the elastic strands of said panel(s) (2, 3) have a dtex that is equal to or greater than, preferably greater than, that of the elastic strand(s) of said transversal waist barrier(s) (5). Preferably the elastic strand(s) of said transversal waist barrier(s) (5) have a dtex that is from 40% to 85%, preferably from 45% to 75%, even more preferably from 50% to 70%, of the dtex of the elastic strands of said panel(s) (2, 3). Advantageously, this allows the barrier(s) to stand up and provide a gasketing effect close to the skin of the wearer without flattening or adding excessive resistance to stretch that would otherwise impact fit and comfort. The tension differential that results entails that a lower tension in the barrier(s) compared to the belt(s) is generated to a degree that upon stretching of the belt(s) the barrier(s) stand-up away from said belt(s).

The tensile stress (N/m) of the entirety of the front and back panels (2, 3), respectively, may be profiled in order to provide the functional benefits of the present disclosure, such as ease of stretch and application, while also maintaining certain force during wear, to prevent the article from sagging after loading. When the elasticity of the front and back panels (2, 3) are provided by a plurality of elastic strands (11) running in the transverse direction, the tensile stress may be adjusted by one or more of the following methods; 1) elongation rate of the elastic strands (11); 2) density (dtex) of the elastic strands (11); 3) longitudinal pitch of multiple elastic strands (11); and 4) effective length of elasticity of the elastic strands (11) in the transverse direction. By elongation, "0% elongation" is meant the original length of the elastic member.

The front and back panels (2, 3) may each be divided into multiple zones spanning in the transverse direction and defined by its location from the distal edge to the proximal edge relative to the percentage of the seam length wherein the distal edge is considered 0% and the proximal edge is considered 100%. The multiple zones may be configured to provide different tensile stress, or different functions to the front and back panels (2, 3), respectively.

In an embodiment, one or more second elastic strands (12) are comprised directly or indirectly between the outer nonwoven layer (10) and the backsheet (7), preferably between a body-facing surface of the outer nonwoven layer (10) and a garment-facing surface of the backsheet (7). An example of an indirect elastic arrangement is the presence of an outer cover layer (7') as described herein (that would generally be interposed between the backsheet (7) and the one or more second elastic strands (12)), and an example of a direct elastic arrangement is the absence of an outer cover layer (7') as described herein. Advantageously this arrangement allows for a simple yet effective elastification of the barrier(s) permitting optimal gasketing effects.

Preferably, the one or more second elastic strands (12) extends along the entire transversal length of the transversal waist barrier(s) (5) and beyond, preferably crossing the imaginary longitudinal axis (y) in a direction substantially parallel to the imaginary transverse axis (x). Preferably the one or more second elastic strands (12) extends from a first position substantially adjacent a seam edge of the outer nonwoven layer (10) to a second position substantially adjacent an opposite seam edge of the outer nonwoven layer (10) with the imaginary longitudinal axis (y) being positioned therebetween, more preferably wherein the one or more second elastic strands (12) extends from said first position to said second position and crosses and/or overlaps the entire transversal waist barrier(s) (5) generally along the imaginary transverse axis (x). Advantageously this allows not only to form a standing barrier upon stretching but further aids better fit on the wearer's front and/or back waist.

In an embodiment, the absorbent core (8) comprises absorbent material selected from the group consisting of superabsorbent polymer particles and/or fibers; and cellulose fibers, preferably wherein said absorbent material is enclosed within a core wrap comprising top and bottom core wrap layers being a same nonwoven layer folded to sandwich the absorbent material therein or distinct nonwoven layers joined together to sandwich the absorbent material therein. The superabsorbent polymer particles and/or fibers may be comprised in an amount of greater than 50%wt, preferably greater than 60%wt, even more preferably greater than 70%wt, most preferably from 75% to 100%wt, by total weight of absorbent material.

In an embodiment, the back panel (3) is wider (generally in a direction substantially parallel to the longitudinal axis y) than the front panel (2) such that a length of the seams is substantially equal to a width (generally in a direction substantially parallel to the longitudinal axis y) of the front panel (2) and less than a width of the back panel (3); or wherein the front panel (2) is wider than the back panel (3) such that a length of the seams is substantially equal to a width of the back panel (3) and less than a width of the front panel (2). Advantageously this may permit comfort as well as improved protection compared to symmetric arrangements.

It is preferred that the backsheet (7) comprises a further outer cover layer (7') on a garment-facing surface thereof, preferably wherein said outer cover layer (7') comprising one or more nonwoven layers, and/or wherein the backsheet (7) comprises a further outer cover layer (7') that is shorter in the longitudinal direction (generally parallel to the longitudinal axis y) than the topsheet (6) and/or backsheet (7) such that at least a portion of an overlap length between the absorbent insert (4) and the panel (2, 3) is free of said outer cover layer (7') and preferably wherein the backsheet (7) is directly joined to, or is in contact with, the panel (2, 3) in said portion of an overlap length between the absorbent insert (4) and the panel (2, 3) free of said outer cover layer (7'). Advantageously this allows for improved softness in areas where needed and yet allow for material savings thus permitting both reduced cost and waste.

The outer cover layer (7') is preferably different from the inner and/or outer nonwoven layers (9, 10) meaning that they may have different properties selected from basis weight (in gsm) and composition. In an embodiment, the outer cover (7') comprises a spunbond nonwoven preferably comprising bicomponent fibers typically selected from the group consisting of polypropylene and polyethylene. The outer cover (7') preferably has a basis weight that is less than the basis weight of the inner and/or outer nonwoven layers (9, 10). The outer cover (7') may have a basis weight of from 8 g/m² (gsm) to 20 g/m² (gsm), preferably from 10 g/m² (gsm) to 15 g/m². Advantageously, the outer cover provides for softness to the touch to the care giver yet, as selected, it limits stress build-up particularly in embodiments with reduced length in the longitudinal direction as described above. Indeed without wishing to be bound by theory, stress build-up in the transition region corresponding to the terminal edge of the outer cover may result in a higher risk of tearing in the absorbent insert to panel joint.

In a preferred embodiment, the absorbent insert (4) is folded-over to form the waist barrier(s) (5) such that at least a portion of the topsheet (6) of the non-folded part of said absorbent insert (4) may come into contact with the folded part of said absorbent insert (4). Advantageously this allows the barrier to overlap the topsheet so as to help guide the exudates therethrough and into the core once stopped by said barrier.

Preferably, the waist barrier(s) (5) extends from 5% to 50%, preferably from 10% to 40%, even more preferably from 15% to 30%, of the total folded length (TFL) of the outer nonwoven layer (10) along the longitudinal axis (y). Advantageously, this may allow formation of a sufficiently large pocket yet avoid excessive resizing of the absorbent insert that may add cost and waste.

In an embodiment, the substantially liquid impermeable film layer or backsheet (7) comprises indicia, preferably a coloured print, at a position corresponding to the transversal waist barrier (5); and/or wherein the one or more second elastic strands (12) are coloured; and/or wherein a garment facing surface of the outer nonwoven layer (10) is coloured; and/or wherein the transversal waist barrier (5) comprises indicia selected from a coloured print and coloured adhesive (preferably comprising one or more pigments). Advantageously this allows the user or care giver to better visualise the barrier(s) so as to aid correct positioning and placement thereof onto a subject.

In an embodiment, the absorbent article further comprising a pair of longitudinally extending cuffs (105) positioned along left and right longitudinal edges of the absorbent insert (4), and wherein the transversal waist barrier(s) (5) is positioned above said cuffs such that said barrier(s) (5) is closer to a wearer's skin than said cuffs; preferably wherein the cuffs are joined to a body-facing surface of the topsheet (6) and wherein each of said cuffs is folded and joined to itself on a body-facing surface thereof at a joining position (JP) corresponding to the transversal waist barrier(s) (5), preferably said joints or joining positions (JP) extending along a lengthwise portion of, and being substantially adjacent to, the left and/or right longitudinal edges of the absorbent insert (4). Advantageously this reduces risk of leakage multidirectionally and prevents leakage to seep through between the transversal barrier and cuffs unlike when otherwise arranged.

Preferably the transversal waist barrier (5) is an up-standing barrier generally arranged to block substantially liquid exudates from flowing therethrough. Preferably, the liquid impermeable film layer is breathable and has a basis weight of less than 50 g/m², preferably from 8 g/m² to 45 g/m². The film may be elastic (or may comprise elastic material such as one or more elastic strands and/or elastic foam) and may comprise micro-openings sized to allow at least some water vapour to permeate therethrough. Preferably the film having a ratio of the MD load at break to the CD load at break of less than about 8, and a machinedirection notched trapezoidal tear strength of at least about 25 g.

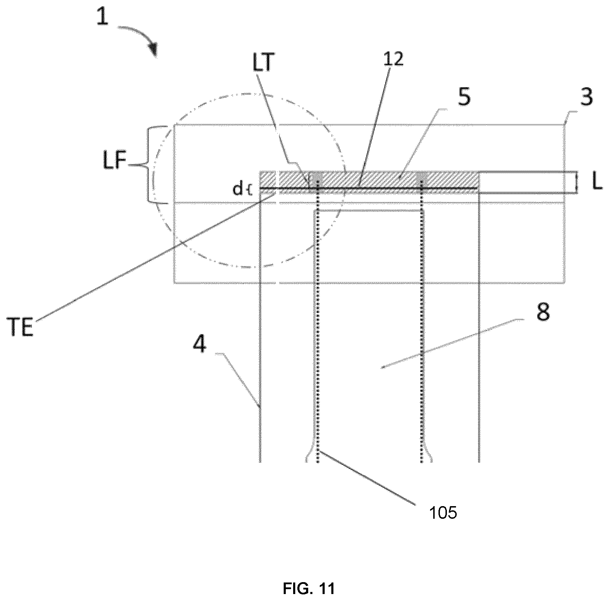

As shown in FIG. 11, in this embodiment, a portion of the absorbent insert proximal (typically adjacent) to the front and/or back transversal edges thereof may be folded onto itself and the fold (as described in embodiments herein) has a length (L) in a longitudinal direction running substantially parallel to a longitudinal axis (y), and wherein said absorbent insert (4) further comprises a pair of longitudinally extending cuffs 105 positioned along left and right longitudinal edges thereof and joined thereto along a tack-down length (LT) extending from a position proximal (preferably adjacent) to at least the back transversal edge to a tack-down terminal position longitudinally displaced therefrom along said longitudinal axis (y), and wherein the tack-down length (LT) is less than or equal to about 2L, preferably said cuffs 105 being joined to a body-facing surface of the topsheet (6). It is understood herein that whilst this embodiment makes specific reference to the "back" transversal edge, when the transversal waist barrier(s) (5) are positioned at the front of the article, the same dimensional characteristics apply with respect to the "front" transverse edge. Advantageously, this allows for a reduced tack-down length that permits to maximise the volume of the containment pocket formed by extending the lateral cavities when the fold stands up in contact with the user when the article is worn.

The back and/or front transversal edge (generally in folded state, as can be generally seen in the figures) typically coincides with a terminal edge (TE) of the transversal waist barrier (5) forming a pocket opening.

Preferably, the transversal waist barrier (5) comprises one or more second elastic strands (12) positioned at a distance (d) from a terminal edge (TE) of the transversal waist barrier (5) forming a pocket opening, and wherein the distance between the elastic closest to said terminal edge is less than about 0.6L, preferably from 0.15L to 0.5L, even more preferably from 0.20 to 0.45L. Advantageously this permits the transversal barrier to more easily stand-up and make a seal with the wearer's skin and preferably yet be sufficiently displaced from the terminal edge to minimize risk of elastic exposure to the skin of a wearer.

The font and/or back panels (2, 3) are elastic and comprise an inner nonwoven layer (9), an outer nonwoven layer (10), and an elastic material (11) sandwiched therebetween, wherein the elastic material is selected from the group consisting of an elastic film and a plurality of elastic strands. Preferably, the outer nonwoven layer (10) is wider than the inner nonwoven layer (9) (generally in a direction substantially parallel to the longitudinal axis y) and is folded over said inner nonwoven layer (9) such that it overlaps the portion of said absorbent insert (4) proximal to the front and/or back transversal edges and generally over a fold length (LF), preferably such that said outer nonwoven layer (10) overlaps both a portion of the backsheet (7) and a portion of the topsheet (6) that remains exposed from said one or more transversal waist barrier(s) (5) such to form a flange (F_{L}). Advantageously this allows to cover the backsheet with nonwoven so that the backsheet does not come into contact with the skin of a wearer which could otherwise result in skin irritations and/or less comfort.

The absorbent insert (4) may comprise a topsheet selected from an embossed nonwoven and/or a carded air-through-bonded nonwoven. The embossed nonwoven may comprise a spunbond nonwoven. Advantageously the topographical surface of such particular nonwoven selection allows for the formation of peaks and slumps that acts to not only slow down the movement of liquid stool towards the transversal edges but further allows for containment ridges in areas overlapping the barrier(s) (5).

Preferably, the thickness of the absorbent insert (4) in an overlap area of said waist barrier (5) (e.g. an area of the absorbent insert (4) overlapping with the waist barrier (5)) is less than the remaining thickness of said absorbent insert (4) (e.g. in areas other than the overlap area); and/or wherein the amount of absorbent material in in an overlap area of said waist barrier (5) is less (preferably at least 5%wt less, preferably at least 10%wt less, even more preferably at least 15%wt less, even more preferably at least 20%wt less) than the amount of absorbent material in other areas of said absorbent insert. Generally said overlap area further overlapping with the front and/or back panels respectively. Advantageously this allows for a larger pocket for exudate collection that synergistically cooperates with the overlapping front and/or back belts.

According to the present disclosure, methods herein for the manufacture of an absorbent article 1 comprise the steps of: providing a front panel 2; providing a back panel 3; providing an absorbent insert 4 comprising front and back transversal edges and left and right longitudinal edges connecting the front and back transversal edges to form a perimeter thereof (and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween); joining said absorbent insert 4 to said front and back panels 2, 3, generally such that at least a portion of the absorbent insert 4 overlaps each of the front and back panels 2, 3 and forms a connection bridge between said front and back panels, said joining step being followed by folding a portion of said absorbent insert 4 proximal to the front and/or back transversal edges thereof onto itself, preferably the fold being in the shape of a substantially U-fold, to form transversal waist barrier(s) 5;; and joining said front and back panels 2, 3 together along a pair of oppositely disposed side seams. Advantageously an absorbent article having transversal barrier(s) as described herein can be attained in a simple yet effective manner.

The methods as disclosed herein comprise a plurality of steps in which the step of joining the absorbent insert 4 to said front and/or back panels 2,3 precedes the step of folding a portion of said absorbent insert 4 proximal to the front and/or back transversal edges thereof onto itself. In other words, said folding step, where a portion of said absorbent insert 4 proximal to the front and/or back transversal edges thereof is folded onto itself, occurs after or following said joining step where said absorbent insert 4 is joined to the front and/or back panels 2,3. Said folding step can occur directly after, meaning sequentially after, or subsequentially, the joining step. The present disclosure also encompasses embodiments with additional steps occurring between said joining step and said folding step, for example a step where adhesive is intermittently applied onto a portion of the absorbent insert 4, a step where said absorbent insert 4 is conveyed, or transported, downstream, for example in the machine direction, from said joining step to said folding step. The present disclosure also encompasses embodiments where the joining step and the folding step are partially concurrent, for example, the portion of said absorbent insert 4 proximal to the front and/or back transversal edges is starting to be fold onto itself even though the absorbent insert 4 is not entirely joined to the front and/or back panels 2,3.

By preceding the folding step, the joining step enables a stabilisation of the absorbent insert 4 before the portion of said absorbent insert 4 proximal to the front and/or back transversal edges thereof is folded onto itself. Advantageously, once joined to the front and/or back panels 2,3, the absorbent insert 4 is stabilized and the portion of said absorbent insert 4 proximal to the front and/or back transversal edges thereof can be folded onto itself more easily.

Generally, the front and back panels herein are made from continuous webs that are subsequently severed into discrete front and back panels typically after the step of joining the absorbent insert 4 to said front and back panels 2, 3 and preferably after the step of folding a portion of said absorbent insert 4 proximal to the front and/or back transversal edges thereof onto itself.

The present disclosure is not limited to this sequence of steps and the present disclosure encompasses different sequence of steps. For example, the present disclosure pertains to a method for the manufacture of an absorbent article 1 comprising the steps of: providing a front panel 2; providing a back panel 3; providing an absorbent insert 4 comprising front and back transversal edges and left and right longitudinal edges connecting the front and back transversal edges to form a perimeter thereof; joining said absorbent insert 4 to said front and back panels 2, 3; joining said front and back panels 2, 3 together along a pair of oppositely disposed side seams and folding a portion of said absorbent insert 4 proximal to the front and/or back transversal edges thereof onto itself, preferably in the shape of a substantially U-fold, to form transversal waist barrier(s) 5. According to another example, the present disclosure pertains to a method for the manufacture of an absorbent article 1 comprising the steps of: providing a back panel 3; providing an absorbent insert 4 comprising front and back transversal edges and left and right longitudinal edges connecting the front and back transversal edges to form a perimeter thereof; joining said absorbent insert 4 to said back panel 3; providing a front panel 2; joining the absorbent insert 4 to the front panel 2; folding a portion of said absorbent insert 4 proximal to the front and/or back transversal edges thereof onto itself, preferably in the shape of a substantially U-fold, to form transversal waist barrier(s) 5 and joining said front and back panels 2, 3 together along a pair of oppositely disposed side seams. In these examples, the step of joining the absorbent insert 4 to said front and/or back panels 2,3 precedes the step of folding a portion of said absorbent insert 4 proximal to the front and/or back transversal edges thereof onto itself.

Logically, to make an absorbent article 1 comprising a transversal barrier 5 arranged in the back, the method comprise a step of folding a portion of said absorbent insert 4 proximal to the back transversal edges thereof onto itself, to make an absorbent article 1 comprising a transversal barrier 5 arranged in the front, the method comprise a step of folding a portion of said absorbent insert 4 proximal to the front transversal edges thereof onto itself and to make an absorbent article 1 comprising two transversal barriers 5 arranged in the front and back, the method comprise a step of folding two portions of said absorbent insert 4 each respectively proximal to the front and back transversal edges thereof onto itself.

Preferably, said folding step is carried out by static folding where a static element, *i.e.* a stator or a stationary element such as a stationary rail or rod or plate or blade, deflects a portion of said absorbent insert 4 proximal to the front and/or back transversal edges thereof onto itself. In other words, the folding is step is preferably done by passive folding meaning that the folding is done with a moving element, *i.e.* the absorbent insert 4, being folded by an unmoving, or immobile or fixed element, e.g. a rail or a rod or a blade. Such folding is more cost efficient and more reproducible. The present disclosure encompasses embodiments where the folding step is carried out by dynamic folding, where a mobile element, e.g. an actuated rail or rod or folding blade, moves or is actuated to fold a portion of said absorbent insert 4 proximal to the front and/or back transversal edges thereof onto itself.

Preferably, the method further comprises the steps of folding an outer nonwoven layer 10 of the front and/or back panels 2, 3 over the folded absorbent insert 4 and/or an inner nonwoven layer 9 and joining said folded outer nonwoven layer 10 directly or indirectly to the folded absorbent insert 4. Advantageously this allows to prevent irritation or discomfort to the wearer as explained hereinabove whilst avoiding the use of added components/materials/elements whilst maintaining a simple and cost-effective production process.

Preferably, the method further comprises the step of arranging an elastic material 12 over the folded portion of the folded absorbent insert 4 prior to step of folding an outer nonwoven layer 10 of the front and/or back panels 2, 3 over the folded absorbent insert 4. Advantageously this allows the transversal barrier(s) 5 to stand more upright.

Preferably, the method further comprises the step of intermittently applying adhesive onto said portion of the absorbent insert proximal to the front and/or back transversal edges. The adhesive can be applied in a uniform application, meaning in an uniform coating, or in swirls or in overlapping spirals. The adhesive can also be applied randomly meaning the fibers of the adhesive material are applied and arranged in a random configuration. For example, the adhesive can be applied using a spray nozzle in a spray nozzle application or the adhesive can be applied using a slot nozzle in a slot nozzle application or the adhesive can be applied using a roller in a roller application or the adhesive can be applied using a track applicator in a track application. Preferably, the adhesive is applied in a uniform spray application using a spray nozzle as such nozzles deliver consistent hot melt adhesive spray coatings, producing uniform patterns and repeatable bonds. The adhesive is applied in joining zones 70 or bonding zones that will be described hereunder. The joining or bonding zones 70 comprises at least one area where the adhesive or mechanical bonding means is/are applied.

According to an embodiment as illustrated in FIG. 4, which represents an absorbent article 1 after the folding step where a portion of said absorbent insert 4 proximal to the back transversal edges thereof is folded onto itself and where the adhesive was applied in the portions 70 located at the corners of the back transversal edge of the absorbent insert 4 but is substantially not applied on the portion where the absorbent core 8 extends.

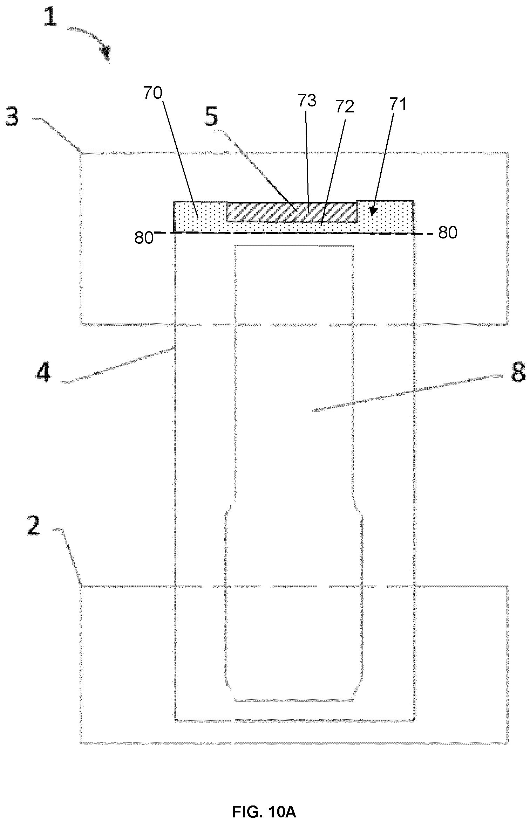

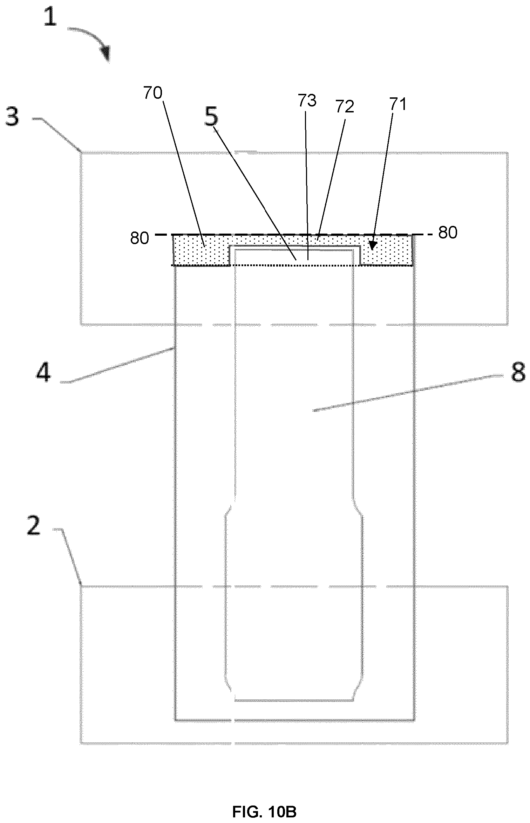

According to another embodiment as illustrated in FIG. 10A, which represents an absorbent article 1 prior to the folding of a portion of said absorbent insert 4 proximal to the back transversal edges thereof onto itself, the adhesive is applied in a pattern 71 on a portion of said absorbent insert 4 proximal to the back transversal edge. FIG. 10B illustrates the same absorbent article 1 after the folding step where the portion of the absorbent insert 4 proximal to the back transversal edge is folded thereof onto itself. The adhesive can also be applied in such pattern 71 to one portion of said absorbent insert 4 proximal to the front transversal edge. Naturally, the adhesive can be applied in such pattern 71 to both portions of said absorbent insert 4 proximal to the front and back transversal edges. The pattern 71 of adhesive comprises the two portions or two areas of adhesive 70, meaning two joining zones, as described hereabove, meaning the area 70 at the transversal extremities of the transversal edge, or in other words, the corners of the absorbent insert 4, or also the portions where the back transversal edge and longitudinal edges of the absorbent insert 4 connect as illustrated in FIG. 10A. The pattern 71 of adhesive further comprises a further area 72 of adhesive extending transversally connecting the two areas 70 and an area free of adhesive 73, said area free of adhesive 73 is arranged between the transversal edge of the absorbent insert 4 and the further area 72 of adhesive with respect to the longitudinal direction (y) and arranged between the two areas 70 at the corners with respect to the transversal direction (x). In other words, the pattern 71 of adhesive comprise a U-shaped pattern with two areas 70 corresponding to the upper branches of the U, said areas 70 being connected by a further area 72 corresponding to the lower branch of the U. Similarly, the pattern 71 of adhesive can comprise a H-shaped pattern with two areas 70 corresponding to the side branches of the H, said areas 70 being connected by a further area 72 corresponding to the middle branch of the H. The pattern of adhesive can comprise two areas 70 at the corners and a further area 72 of adhesive which extend only partially along the transversal axis (x) and does not connect the two areas 70 while still defining an area free of adhesive 73 as described above. In other words, the further area 72 can comprise a gap free of adhesive. In other words, the pattern 71 of adhesive can comprise two areas 70 that are L shaped in facing relationship or in mirror relationship, said L-shaped areas being arranged at each corner.

With an adhesive pattern 71 as described hereabove, when folding the absorbent insert 4 along the folding line 80 illustrated in FIG. 10A or FIG. 10B, a transversal waist barrier 5 is defined with the area free of adhesive 73 not being joined to the absorbent insert 4 and therefore standing upright. In other words, the barrier(s) 5 herein form an opening, typically about a terminal edge of the folded outer nonwoven layer, of an exudate containing pocket wherein substantially the entire surface of the pocket being congruent with a topsheet 6 of the absorbent insert 4 comprises a liquid impermeable layer preferably being a film, more preferably an elastic film (generally according to the embodiments described herein). With such adhesive pattern 71, the angle defined by the fold and the waist barrier is more acute and can provide a transversal waist barrier 5 with more resistance to exudates.

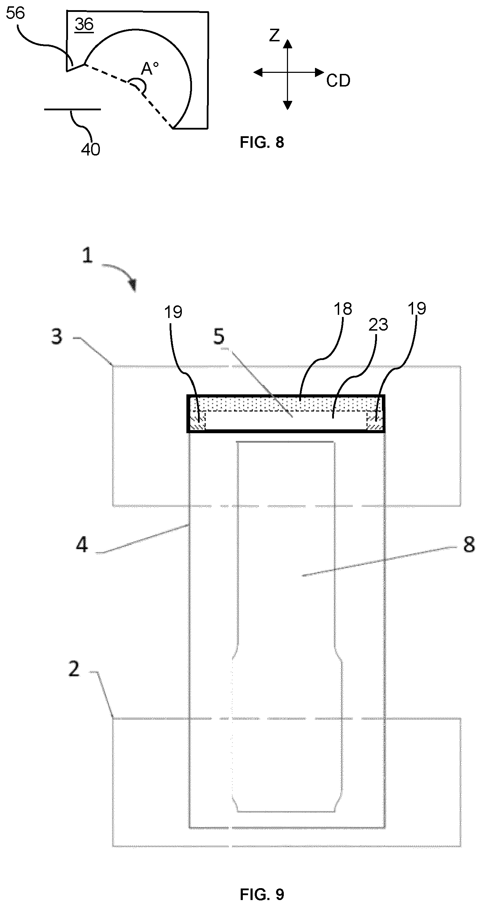

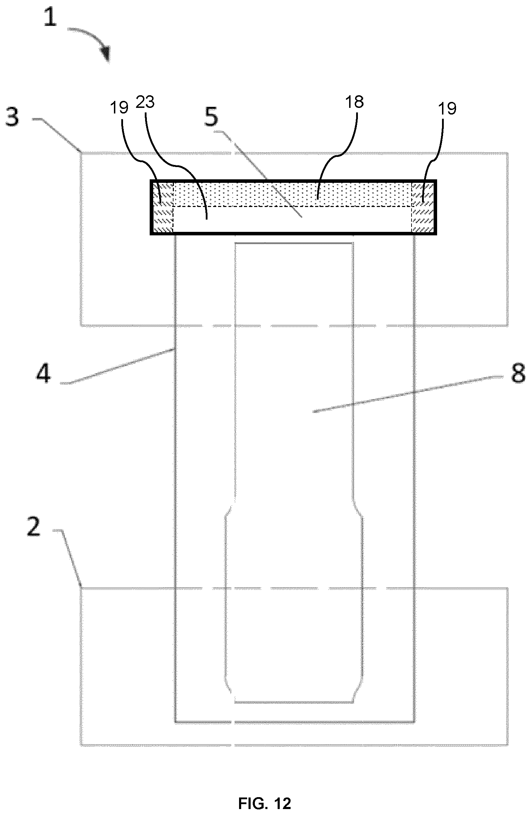

According to an embodiment, the method can comprise a mechanical bonding step where the portion of the absorbent insert 4 proximal to the back transversal edge is folded thereof onto itself and then mechanically bonded. The method comprises a step where the portion of the absorbent insert 4 proximal to the back transversal edge is glued to the absorbent insert 4 and/or a step where the portion of the absorbent insert 4 proximal to the back transversal edge is mechanically bonded to the absorbent insert 4. For example, as illustrated in FIG. 9 or FIG. 12, adhesive is intermittently or continuously applied to a central portion 18 of the absorbent insert 4 proximal to the back transversal edge, then said portion is folded thereof onto itself, then the folded portion is mechanically bonded in portions 19 arranged at the lateral portion, meaning at each longitudinal ends, of said portion proximal to the transversal edge, e.g. by ultrasonic welding, to further secure the transversal waist barrier 5, the unbonded portion 23 serving as the transversal barrier 5.

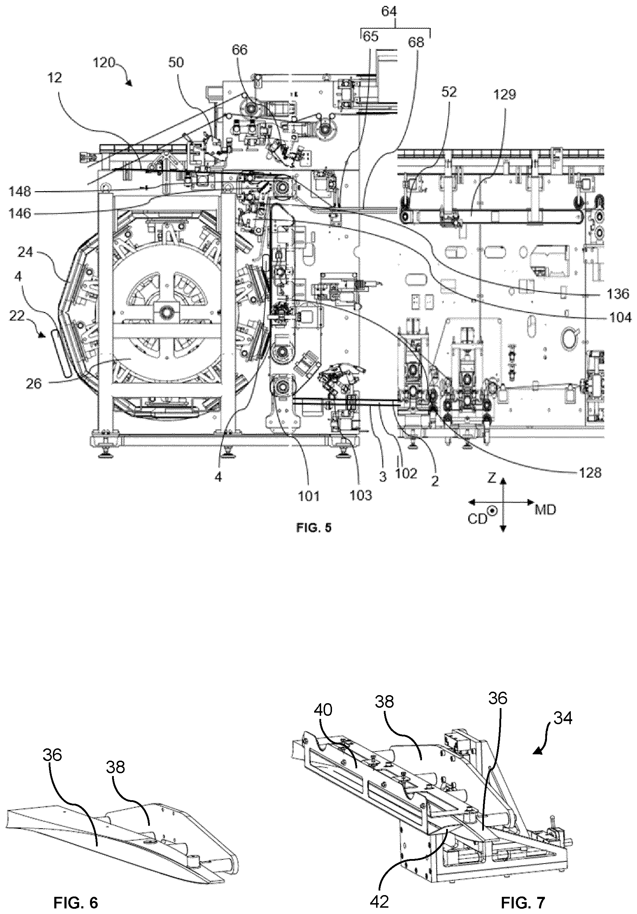

The present disclosure also pertains to an apparatus 120 to manufacture an absorbent article 1 as described herein and following the methods disclosed herein. Figure 5 illustrates one possible embodiment for an apparatus 120 for manufacturing such absorbent articles 1 by carrying out a method as disclosed herein. The apparatus 120 extends in a machine direction MD, a cross-direction CD and a vertical direction Z. The machine direction MD corresponds to the direction of travel of the webs, elastic materials or any components of the absorbent articles 1 being manufactured, the cross-direction CD is a direction perpendicular to the machine direction MD, and the vertical direction Z correspond to the height and is also a direction perpendicular to both the machine direction MD and the cross-direction CD. In other words, the machine direction MD is the flow direction of an absorbent article and its components being assembled in the process line, e.g. the machine direction MD is the direction in which the absorbent insert 4 is conveyed. The terms "upstream" and "downstream" as used herein are with respect to the machine direction MD.

For sake of clarity, the coordinate system using longitudinal and transversal axis (x,y) pertains to the absorbent article 1 alone e.g. when laid flat on a surface, whereas coordinate system using machine, cross and vertical directions (MD, CD, Z) pertains to the apparatus for manufacturing absorbent article 1 with the absorbent articles 1 and their components being conveyed.

Following the methods described herein, an absorbent insert 4 is provided. Here, the absorbent insert 4, assembled beforehand, is transferred by a transfer device 22, a unit 22 comprising a plurality of transfer heads 24 arranged on a support wheel 26 with both the support wheel 26 and the transfer heads 24 being configured to respectively rotate around a respective axis. The transfer device 22 is an apparatus that is configured to turn and repitch the discrete absorbent inserts 4, turn the discrete absorbent inserts 4, or merely transfer the discrete absorbent inserts 4 between a pick-up location and a drop-off location. The transfer device 22, as illustrated in FIG. 5, comprises a frame 26, here a support wheel 26, defining a rotation axis and a plurality of transfer heads 24 associated to said frame 26. The transfer heads 24 are configured to circumnavigate about the rotation axis in an orbit. As illustrated in FIG. 5, the support wheel 26 rotates around an axis parallel to the cross-direction CD and the transfer heads 24 circumnavigate about this rotation axis in an orbit. The orbit passes through the pick-up location and the drop-off location. The transfer device 22 turns the discrete absorbent inserts 4 in any suitable angle. For example, as illustrated in FIG. 5, the transfer device 22 turns the discrete absorbent inserts 4 about 90 degrees. The discrete absorbent inserts 4 are turned by the transfer heads 24 turning. For instance, a transfer head 24 may pick up a discrete absorbent insert 4 in a pick-up location, turn 90 degrees about an axis perpendicular to the rotation axis of the frame, e.g. about the vertical direction Z, drop off the discrete absorbent insert 4 in a drop-off location, and the turn back to its original position (either in the same direction or an opposite direction) before orbiting back through the pick-up location. In other words, the transfer device 22 transfers an absorbent insert 4 extending in the machine direction (MD) and rotates it so that said absorbent insert 4 extends in the cross direction (CD). Meaning before the transfer device 22 the longitudinal axis y of the absorbent insert 4 extends in the machine direction (MD) whereas after the transfer device 22 the longitudinal axis y of the absorbent insert 4 extends in the cross direction (CD).

A front panel 2 and a back panel 3 comprising the inner nonwoven layer 9 and outer nonwoven layer 10 as described hereabove, are conveyed, for example by a rotating roller 101, towards a first conveying device 128, e.g. a conveyor belt 128. The front and back panels 2,3 are assembled beforehand with the lamination of inner and outer nonwoven layers 9,10 and elastic material 11 and by intermittently severing the elastic material 11. The front and back panels 2,3 are for example conveyed with a gap 102 arranged in between them, in other words, the front and back panels 2,3 are separated by a gap 102 in the cross direction (CD) or in other terms, the front and back panels 2,3 are conveyed in the machine direction (MD) and/or vertical direction (V) and are at a distance of one another with respect to the cross direction (CD). The gap 102 as illustrated in FIG. 4, can correspond for example to the distance separating the front and back panels 2,3 in the absorbent article 1 for example when laid flat on a surface and with respect to the longitudinal axis y. The gap 102 is lesser than the distance defined by the front and back transversal edges of the absorbent insert 4 with respect to the longitudinal axis y. According to an embodiment, the front and back panels 2,3 are assembled together, meaning as one web, and are cut and separated prior to being joined to the absorbent insert 4 either individually or simultaneously.

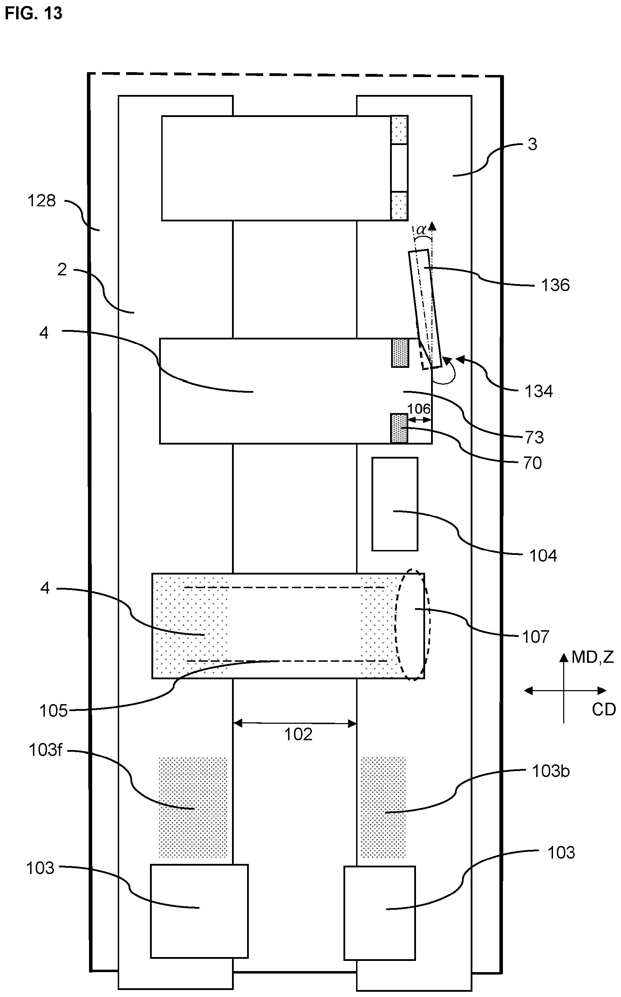

The front and back panel 2,3 are conveyed towards a first adhesive dispensing device 103 to apply adhesive on the portion of the front and/or back panels 2,3. Specifically, the first adhesive dispensing device 103 applies adhesive on the portion of the front and/or back panels 2,3 meant to be in contact with the absorbent insert 4. For embodiments where the absorbent article 1 comprises only one transversal barrier 5, either in the front or back, the first adhesive dispensing device 103 applies, or dispenses, a greater amount of adhesive onto one panel than onto the other panel. For example, for an absorbent article 1 comprising a transversal barrier 5 arranged in the back, the first adhesive dispensing device 103 preferably applies a greater amount of adhesive onto the front panel 2 than onto the back panel 3, for example as illustrated in FIG. 13 the surface area 103b of adhesive applied onto the back panel 2 is lesser than the surface area 103f of adhesive applied onto the front panel 2. The first adhesive dispensing device 103 comprise at least one nozzle to selectively apply adhesive on both panels 2,3, preferably two nozzles, one for each panel 2,3.

The front and back panels 2,3 are conveyed towards a first conveying device 128, meaning the front and back panels 2,3 are deposited onto the conveying device 128, preferably a conveyor belt. As illustrated in FIG. 13, the first adhesive dispensing device 103 can apply adhesive onto the front and back panels 2,3 following said panels 2,3 being conveyed by the first conveying device 128 or as illustrated in FIG. 5, the first adhesive dispensing device 103 can apply adhesive onto the front and back panels 2,3 prior to said panels 2,3 being conveyed by the first conveying device 128. As illustrated in FIG. 5, the first conveying device 128 extends in the vertical direction, meaning that the front and back panels 2,3 are guided or conveyed in the vertical direction Z, in other words, for this portion of the process the machine direction MD, corresponding to the direction of travel of these two webs 2,3, is in the same direction as the vertical direction Z or in other terms for that portion of the process the machine and vertical directions MD, Z are the same. The first conveying device 128 extends in the vertical direction Z, such arrangement enables to gain space in the process line.

The absorbent insert 4 is deposited onto the first conveying device 128, e.g. conveyor belt 128 and onto the front and back panels 2,3 being conveyed by the first conveying device 128. The first conveying device 128 is associated with a negative pressure device such that the front and/or back panels 2,3 and absorbent insert 4 when deposited onto the conveying device 128 are sucked and maintained onto the outer surface of the conveying device 128 by vacuum. Preferably the first conveying device 128 comprises vacuum holes on its outer surface, meaning a foraminous outer surface, and the front and back panels 2,3 and absorbent insert 4 are deposited onto said outer surface. The front and back panels 2,3 as well as the absorbent insert 4 are maintained on the foraminous first conveyor device 128 by the negative air-pressure generated by the suction. As illustrated in FIG. 5 and FIG. 13, the absorbent insert 4 is deposited onto the front and back panels 2,3 conveyed by the conveying device 128, as well as onto the conveying device 128 in the gap 102 region. More specifically, the absorbent insert 4 is deposited onto the surface areas 103b,103f of adhesive applied onto the front and back panel 2,3 by the first adhesive dispensing device 103. The absorbent insert 4 is hence maintained, or stabilized, by the combination of vacuum in the gap 102 region and by the adhesive present on the front and back panel 2,3.

The front and back panels 2,3 and the absorbent insert 4 are conveyed towards a second adhesive dispensing device 104 to apply adhesive on said absorbent insert 4. Said adhesive is applied, preferably intermittently, on at least one portion of said absorbent insert 4 proximal to the front and/or back transversal edges. As illustrated in FIG. 13, the adhesive dispensing device 104 comprises one nozzle and applies adhesive intermittently onto the portion of said absorbent insert 4 proximal to the back transversal edge. In other words, when being conveyed by the first conveyor belt 128, adhesive is applied intermittently onto the absorbent insert 4 by the second adhesive dispensing device 104 on at least one portion proximal to a transversal edge, namely the adhesive is applied intermittently on a portion of said absorbent insert 4 proximal to the front and/or back transversal edges, meaning on the portion described hereabove to be folded onto itself. In other words, the adhesive is applied intermittently on at least one edge arranged at one transversal end of the absorbent insert 4 with respect to the cross-direction CD as illustrated in figure 13. The adhesive can be applied intermittently on one portion of said absorbent insert 4 proximal to the back transversal edges. The adhesive can be applied intermittently to one portion of said absorbent insert 4 proximal to the front transversal edges. The adhesive can be applied intermittently to both portions of said absorbent insert 4 proximal to the front and back transversal edges. The adhesive is applied intermittently such that the central area of said portion(s) proximal to the transversal edge(s) is not glued. In other words, the adhesive is applied on both portions 70 arranged at, or near, the corners of a transversal edge, but not in between said corners 70 as illustrated in FIG. 4. For example, when considering FIG. 4, the adhesive is applied in the portion 70 located at the corner of the back transversal edge but is not applied on the portion where the absorbent core 8 extends, meaning when the back transversal edge and the absorbent core are transposed on a same machine direction axis, the adhesive applied is present at the corners but not where the absorbent core 8 extends. According to one embodiment, the adhesive is applied intermittently on one transversal edge only, preferably the back transversal edge. According to another embodiment, the adhesive is applied intermittently on both transversal edges. As illustrated in FIG. 13, the adhesive dispensed by the second adhesive dispensing device 104, is applied at the portion of the absorbent insert 4 proximal to the back transversal edge, at each longitudinal edge said portion proximal to the back transversal edge and preferably at a distance 106 from said transversal edge so as to not hinder, or obstruct or make difficult, the proper folding of said portion onto itself. Alternatively, the adhesive can also be applied continuously in an adhesive pattern 71 as described above, for example in a U-shaped or a H-shaped pattern.

The adhesive is applied by the second adhesive dispensing device 104. Such application of adhesive may be by various processes such as slot coating, spray coating and other topical applications using a pneumatic, piezo or electromagnetic actuator. For example, the second adhesive dispensing device 104 comprises at least one nozzle, each nozzle, for instance, may comprise a slot coating device or several exit ports and an adhesive flow control device such as electromagnetic valve, e.g. a solenoid valve. Similarly as for the first application of adhesive applied by the first adhesive dispensing device 103, the second application of adhesive applied by the second adhesive dispensing device 104 can be applied in a uniform application, meaning in an uniform coating, or in swirls or in overlapping spirals. The adhesive can also be applied randomly meaning the fibers of the adhesive material are applied and arranged in a random configuration. For example, the adhesive can be applied using a spray nozzle in a spray nozzle application or the adhesive can be applied using a slot nozzle in a slot nozzle application or the adhesive can be applied using a roller in a roller application or the adhesive can be applied using a track applicator in a track application. Preferably, the adhesive is applied in a uniform spray application using a spray nozzle as such nozzles deliver consistent hot melt adhesive spray coatings, producing uniform patterns and repeatable bonds. For example with an adhesive pattern 71 comprising two separate, or distinct or unconnected, areas of adhesive 70, e.g. arranged proximal to the corners of the back transversal edge of the absorbent insert 4 or the two L-shaped areas in facing relationship, a spray nozzle may be more adapted. With an adhesive pattern 71 comprising a continuous application of adhesive, such as a U-shaped or H shaped pattern, a slot nozzle may be more adapted.

The absorbent insert 4 is then conveyed, or transported, by the first conveying device 128 to a folding unit 134. The folding unit 134 functions as following, the absorbent insert 4 is joined to the front and/or back panels 2,3, namely at the surface areas 103f,103b of adhesive as described hereabove, and is maintained in position onto the first conveying device 128 by said adhesive as well as the vacuum, or negative air-pressure, maintaining the front and/or back panels 2,3 onto the conveying device 128. As mentioned above, the absorbent insert 4 usually comprises a pair of longitudinally extending cuffs 105 which are elastic elements, such as elastic strands, extending longitudinally in the absorbent insert 4, said elastic elements being often referred as leg cuffs 105. These cuffs 105, being stretched elastic elements, continuously try to revert to their un-stretched configuration and hence induce a retraction force, or a tension force or an elastic force or a kinetic force, onto the absorbent insert 4. The absorbent insert 4, not being entirely joined to the back panel 3, meaning the portion(s) of the absorbent insert 4 proximal to the front and/or back transversal edges that is/are not glued - see for example reference 107 in FIG. 13, start inducing a movement of folding onto itself. More specifically, as exemplified in the embodiment of FIG. 13, the portion 107 of the absorbent insert 4 proximal to the back transversal edge thereof, not being glued and under the elastic tension of the cuffs 105, said portion 107 starts being pulled towards the centre of the absorbent insert 4 or in other words said portion 107 starts folding onto itself. Whilst such embodiment is advantageous as it requires no additional means to start inducing a folding step, the present disclosure is not limited to this embodiment as there can be other means to induce a folding step. For example, the first conveying device 128 can comprise a hole to blow out compressed air to lift said portion 107 or the apparatus 120 can comprise a folding blade 136 with a pressing blade 40 as described hereunder.

As explained above, the absorbent insert 4 is conveyed, or transported, by the first conveying device 128 to a folding unit 134. The folding unit 134 preferably comprises a static element 136, meaning an element that is stationary and does not move such as a rail or a rod or a blade. In order to accelerate and improve the folding, the folding unit 134 comprises a static element 136 to further force said portion 107 of the absorbent insert 4 proximal to the back transversal edge thereof to fold onto itself. In other words, the static element 136 deviates the portion 107 of the absorbent insert 4 proximal to the back transversal edge thereof and facilitate and enhance the folding motion so that the portion 107 of the absorbent insert 4 proximal to the back transversal edge thereof can effectively fold onto itself. In other words, the portion 107 of the absorbent insert 4 proximal to the back transversal edge thereof when starting to be pulled towards the centre of the absorbent insert 4 by the retraction force, or elastic force, induced by the cuffs 105, is further deflected by the static element 136 which further promote or accelerate the folding movement or motion. Additionally, such static element 136 enables to have a smoother fold and reduces the risks of wrinkles or a gap in the fold.

The static element 136 can extend in the machine direction (MD) or the static element 136 can extend in both the machine direction (MD) and cross direction (CD) as illustrated in FIG. 13 or the static element 136 can extend in the machine direction (MD) and cross direction (CD) and/or vertical direction (Z).

For example, as illustrated in FIG. 13 the static element 136 extends in both the machine direction (MD) and cross direction (CD), meaning diagonally with respect to the machine direction (MD). With this arrangement, the stator 136 can further promote the deflection of the portion of the absorbent insert 4 proximal to the back transversal edge thereof towards the centre and thus the folding of the portion of the absorbent insert 4 proximal to the back transversal edge thereof onto itself. For example the static element 136 extends diagonally, meaning the static element 136 is elongated in a longitudinal axis, said axis defining an angle *α* with respect to the machine direction (MD), said angle *α* is comprised between 1° and 45°, preferably between 2° and 30°, more preferably between 3° and 15°, 90° corresponding to the cross direction (CD), as illustrated in FIG. 13, the static element 136 extends diagonally with an angle *α* of 8° with respect to the machine direction (MD).

The static element 136 extends along the machine direction (MD) with a length that is lesser or equal or greater than the total transversal length (generally substantially parallel to the transversal axis x) of the absorbent insert 4. Preferably, the length of the static element 136 is equal or greater than the total transversal length of the absorbent insert 4 with respect to the machine direction (MD) to ensure that the deflection is applied all along the portion 107 of the absorbent insert 4 proximal to the back transversal edge thereof. However given that the absorbent insert 4 is conveyed by the first conveying device 128 along the machine direction (MD), even if the length of the static element 136, with respect to the machine direction (MD), is lesser than the total transversal length of the absorbent insert 4, the static element 136 can still promote the folding movement or motion induced by the cuffs 105.

According to an embodiment, the static element 136 is arranged at a distance from the first conveying device 128 and this distance can be constant or can vary. For example, the distance between the static element 136 and the first conveying device 128 can decrease along the machine direction so that at the downstream portion of the static element 136, the static element 136 and the first conveying device 128 form a nip to ensure a proper folding of the portion 107 of the absorbent insert 4 proximal to the back transversal edge thereof onto itself.

The static element 136 can comprises for example a folding rail 136, a folding rod 136, a folding plate 136 or a folding blade 136. The folding rail 136 or folding rod 136, as illustrated in FIG. 13 extends linearly, meaning along one direction in the machine direction (MD) and eventually in the cross direction (CD). For example, the static element 136 can be a folding rail 136, or rod, meaning a thin straight bar, for example made out of metal, or the static element can be a folding plate 136, meaning a smooth flat piece of material, preferably metal. The folding rail 136, rod or plate can also be curved. The folding rail 136, rod or plate can also be connected to an air-compressor and comprise air-vents or holes on its surface to blow out pressurized air to further promote the folding of the portion of the absorbent insert 4 proximal to the back transversal edge thereof onto itself.

The folding unit 134 can comprise a folding blade 136 that is curved (FIG. 6,9) and that can be associated with a support plate 38 as illustrated in FIG. 6. The folding blade 36, or curved blade 36, comprises a blade with a curvature said curvature spanning over an angle from 0° to at least 200°. In other words, the curved blade comprises a concave portion, said concave portion varying along the machine direction and extending over an angle A° of at least 180°, preferably at least 250°, more preferably at least 300°, meaning angle A° is preferably a reflex angle as illustrated in FIG. 8. For sake of clarity, the folding blade 36 comprises a curve blade 36, said curved blade 36 defines an angle A° that evolves along the machine direction MD, meaning that for a given point in the machine direction MD, the folding blade 36 extends in the vertical and cross directions Z,CD as illustrated in FIG. 8, the two extremities of said blade 36 defining an angle A°, or folding angle A°, since the folding wall, meaning the wall used for folding the absorbent insert 4, of the blade is curved. In the upstream portion of the folding blade, the angle A° defined by the two extremities of the blade 36 is small, e.g. between 0° and 10°. The angle A° increases along the machine direction MD and is greater than 180° at the downstream portion of the folding blade 36. The support plate 38 is fixed, or removably associated to the folding blade 36. Having a blade 36 with a folding angle A° of 180° is sufficient to fold the absorbent insert 4 onto itself but it can lead to having wrinkles or a gap in the fold. Having a blade 36 with a folding angle A° of 250° or more enables to have a smoother fold and reduces the risks of wrinkles or a gap in the fold. said absorbent insert

The folding unit 34 can further comprise a pressing blade 40 arranged at a distance d from the curved blade 36 with respect to the cross-direction CD and/or vertical direction Z as illustrated in FIG. 8. In other words, there is a gap between the curved blade 36 and the pressing blade 40 in the cross-direction CD and/or vertical direction Z. The pressing blade 40 presses vertically against the absorbent insert 4 and maintain said absorbent insert 4 in place, at least vertically. The combination of the curved blade 36 folding the portion of the absorbent insert 4 proximal to the front and/or back transversal edge, and the pressing blade 40 pushing the absorbent insert 4 at least vertically, ensures a proper folding of the portion of the absorbent insert 4 proximal to the front and/or back transversal edges thereof onto itself, in order words a proper folding of the portion of the absorbent insert 4 proximal to the front and/or back transversal edge to form a transversal barrier 5 as described herein. In other terms, the folding blade 36 deviates the portion of the absorbent insert 4 proximal to the front and/or back transversal edge in the cross direction CD and in the vertical direction Z and the pressing blade 40 deviates a portion of the absorbent insert 4, which is more distal to the transversal edge than said portion of the absorbent insert 4 proximal to the transversal edge e.g. in between said portion of the absorbent insert 4 proximal to the front and/or back transversal edge and the centre of absorbent core 8, in the vertical direction Z. Simply put, the pressing blade 40 at least maintains the absorbent insert 4 in place while the folding blade 36 is folding the portion of the absorbent insert 4 proximal to the transversal edge. The folding blade 36 alone can suffice to fold the portion of the absorbent insert 4 proximal to the front and/or back transversal edge, and adding a pressing blade 40 further improves the folding. The folding blade 36 ensures a folding preferably in the shape of a substantially U-fold, to form transversal waist barrier(s) 5. In the embodiment described above where adhesive is applied intermittently at the corners 70, then only the corners 70 of the portion of the absorbent insert 4 proximal to the front and/or back transversal edges are folded and glued thereof onto itself.

According to an embodiment, in order to properly fold the portion of the absorbent insert 4 proximal to the front and/or back transversal edge thereof onto itself, the pressing blade 40 is preferably shorter in length, with respect to the machine direction MD, than the folding blade 36 so that when the curved blade 36 is folding the portion of the absorbent insert 4 proximal to the front and/or back transversal edge, namely once said portion has been folded by an angle of more than 180°, it folds thereof onto itself, meaning onto the upper surface of the absorbent insert 4, i.e. the topsheet 6.

According to an embodiment, the pressing blade 40 can be a flat plate as illustrated in FIG. 7, or it can be a rod or a cylindric tube. The pressing blade 40 can comprise an anti-adhesive coating to ensure that the absorbent insert 4 when being folded does not adhere onto the pressing blade 40. The pressing blade 40 can also be connected to an air-compressor and comprise air-vents or holes on its surface to blow out pressurized air to ensure that the absorbent insert 4 when being folded does not adhere onto the pressing blade 40.

According to an embodiment, the pressing blade 40 can comprise a sloped portion 42 at the upstream portion of the pressing blade 40 with respect to the machine direction MD as illustrated in FIG. 7. The slope portion 42 extends diagonally downward while extending downstream for holding down the absorbent insert 4 and improving the folding as described hereabove.

Once the at least one portion of the absorbent insert 4 proximal to front and/or back transversal edge has been folded thereof onto itself thereby forming a transversal waist barrier 5, the absorbent insert 4 and front and back panels 2,3 can be conveyed toward one or more pressure roller(s) 146 which is arranged to press the folded portion of the absorbent insert 4, meaning the transversal waist barrier(s) 5, thereby securing the fold and eventually securing the glued and/or welded portions together.

The folded absorbent insert 4 and front and back panels 2,3 are then guided, for example by the first conveying device 128 to an elastic depositing unit or station, where a second elastic material 12, e.g. one or more elastic strands 12, is deposited onto the backsheet 7 or the outer cover 7' of the absorbent insert 4 at the region that has been folded over. The elastic strand(s) 12 is beforehand coated with adhesive by an adhesive dispensing unit 50 such that when the elastic strand(s) 12 is applied on the backsheet 7 or outer cover 7' on the portion of the absorbent insert 4 that has been folded thereof onto itself, it remains in place. The adhesive is preferably continuously applied on the elastic strand(s) 12. In other words, the elastic strands 12 in any of the embodiments herein may be strand-coated with adhesive prior to joining to one or more layers such as the inner and outer nonwoven layers 9, 10 to form elastic panels 2, 3 and/or the backsheet 7, outer cover 7' and/or outer nonwoven layer 10 to form the elastic transversal waist barrier(s) 5.

Preferably the elastic material 12, e.g. the elastic strand(s) 12, is arranged such that said elastic material 12 is sandwiched between the absorbent insert 4 and the outer nonwoven layer 10. More preferably, the absorbent insert 4 as described herein comprises a backsheet 7 and eventually an outer cover 7' on the garment facing surface of the absorbent core 8. The present disclosure encompasses embodiments where the elastic material 12 is sandwiched between the backsheet 7 and the outer nonwoven layer 10, embodiments where the elastic material 12 is sandwiched between the outer cover 7' and the outer nonwoven layer 10 or even embodiments where the elastic material 12 is sandwiched between the backsheet 7 and outer cover 7' and the outer nonwoven layer 10 e.g. in cases where the outer cover 7' is transversally shorter than the backsheet 7. In other words, the elastic material 12 is directly or indirectly joined to the backsheet 7, preferably by adhesive, and sandwiched between the backsheet 7 and/or outer cover 7' and the outer nonwoven layer 10.

The present disclosure is not limited to this sequence of step, for example, the folding step can precede the application of adhesive onto the absorbent insert 4, meaning the folding unit 134 can be arranged upstream of the second adhesive dispensing device since the adhesive is applied at a distance 106 from the transversal edge. According to another example the folding step and the application of adhesive onto the absorbent insert 4 are substantially concurrent, meaning carried out at the same time or point with respect to the machine direction (MD).

As illustrated in FIG. 5, the folded absorbent insert 4 and front and back panels 2,3 are guided from the folding unit 134 to a rotating drum 148 where the folded absorbent insert 4 and front and back panels 2,3 are pivoted, meaning where the machine direction (MD) and the vertical direction (Z) become distinct.

According to an embodiment, the elastic material 12 is deposited onto the backsheet 7 or the outer cover 7' of the absorbent insert 4 at the region that has been folded over at the level of the rotating drum 148. In other words, the elastic material 12 is deposited onto the backsheet 7 or the outer cover 7' of the absorbent insert 4 at the region that has been folded over when the folded absorbent insert 4 and front and back panels 2,3 are being conveyed or transported by the rotating drum 148.

As explained above, the apparatus 120 can comprise one or more pressure roller(s) 146 to secure the fold. Once the at least one portion of the absorbent insert 4 proximal to front and/or back transversal edge has been folded thereof onto itself thereby forming a transversal waist barrier 5, the absorbent insert 4 and front and back panels 2,3 can be conveyed toward one or more pressure roller(s) 146 which is arranged to press the folded portion of the absorbent insert 4, meaning the transversal waist barrier(s) 5, thereby securing the fold and eventually securing the glued and/or welded portions together. The pressure roller 146 and the rotating drum 148 can form a nip where the portion of the absorbent insert 4 proximal to front and/or back transversal edge has been folded thereof onto itself passes through.

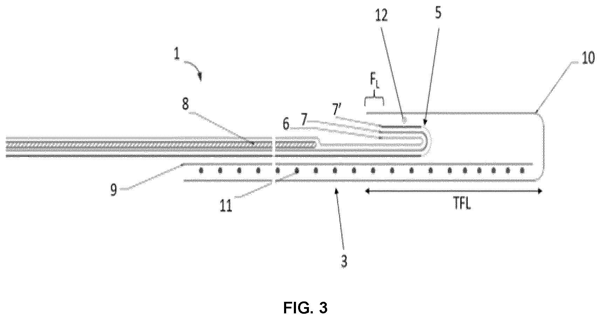

Once the elastic material 12 has been placed onto the backsheet 7 or the outer cover 7' of the absorbent insert 4 at the region that has been folded over, the outer nonwoven layer 10 is then folded over the elastic strand 12, backsheet 7, outer cover 7' and/or the transversal elastic barrier 5 and by a second folding apparatus 64, in order to form the absorbent article 1 as illustrated in FIG. 3. The apparatus 120 can further comprise an additional adhesive dispensing device 66 to apply adhesive on the portion of the front and/or back panels 2,3 to be folded. The apparatus 120 can further comprise a pressure roller 52 to apply pressure on the transversal edges of the front and/or back panels 2,3 and ensure the structural integrity of the chassis. Preferably the outer nonwoven layer 10 is folded over the absorbent insert and/or thereof onto itself by the second folding apparatus 64 at the front and back panels 2,3 and the additional adhesive dispensing device 66 applies adhesive on a portion of the absorbent insert 4 proximal to the transversal edges of the front and back panels 2,3. The second folding apparatus 64 comprises a roller 65 that is arranged to cover a portion of the front and/or back panels 2,3, namely the roller is arranged at a distance from the transversal edge of the front and/or back panel 2,3 so that said roller deviates vertically said portion of the front and/or back panels 2,3, the transversal edge of the front and/or back panel which is not deviated by the roller naturally folds upward. The folding apparatus 64 can further comprise a railing 68 oriented diagonally with respect to the cross-direction CD toward the centre of absorbent insert 4 to guide and facilitate the folding of the non-deviated portion of the outer nonwoven layer 10, i.e. the transversal edge of the front and/or back panel 2,3 onto the absorbent insert 4, namely onto the transversal barrier 5, backsheet 7 and/or outer cover 7'. The web or absorbent articles 1 is conveyed in this portion of the process by a second conveying device 129 such as a second conveyor belt 129.

The belt, meaning the front and back panels 2,3 are then cut in the cross-direction CD into individual pieces, each individual piece comprising an absorbent insert 4 with a front and back panel 2,3. Each individual piece is then folded to bring the front and back panels 2,3 in proximity of one another and then associated in side seams to form an individual absorbent article 1 with a transversal barrier 5. The front and back panels 2,3 are joined, or associated, by bonding said panels 2,3 together at a bonding unit. The bonding unit can be selected from and not limited to an adhesive dispensing device, an ultrasonic device or a welding device. Alternatively, the front and back panel 2,3 can be joined first and then cut in the cross direction into individual pieces.

According to an embodiment, the folding blade 36 comprises an extension 56 as illustrated in FIG. 8 where the extension 56 extends beyond the curvature of the blade 36 to ensure that the transversal edge is well folded thereof onto itself.

According to an embodiment, the first conveyor belt 128 and the second conveyor belt 129 can transport the absorbent inserts 4 and front and back panels 2,3 at the same speed to avoid the formation of wrinkles or creases. The first conveying device 128 and the transfer device 22 can also transport the absorbent inserts 4 at matching speed to avoid the formation of wrinkles or creases.

In order to ensure a good vacuum, the different conveying units comprising the first conveying device 128 and/or the second conveying device 129 can all be linked to one main pump, or central pump, via one channel or via different channels, meaning these conveying units can be fluidically connected. These different conveying units can also be connected to a booster pump that is arranged in-between said conveying unit(s) and main pump, said booster pump being fluidically connected to the main pump. The volume flow rate of the main pump is at least twenty times greater than the volume flow rate of any of the booster pumps. For example, the volume flow rate of the main pump is comprised between 20 000 m³/h and 50 000 m³/h and the volume flow rate of any booster pump is comprised between 100 m³/h and 1 000 m³/h. The main pump and the booster pump(s) are a mean to move fluid, namely air eventually charged with absorbent material, it can be for example a centrifugal fan. Preferably, a booster pump is arranged between the first rotating drum 30 and the main pump, since the vacuum force VF(4)₃₀ is preferably greater than the other vacuum forces. The main pump has preferably a higher volume flow rate, or air capacity, then any of the booster pumps. In other words, the main pump conveys a greater volume flow than any of the booster pumps, *i.e.* the flow of air passing through the main pump is greater than the flow of air passing through any of the booster pumps. The volume flow rate, or air capacity, of the main pump 38 is at least twenty times greater than the volume flow rate, or air capacity, of any of the booster pumps. Preferably, the volume flow rate, or air capacity, of the main pump is at least twenty times, more preferably between 30 and 250 times greater than the air capacity of any of the booster pumps, more preferably between 35 and 200 times greater. The main pump and each of the booster pumps are preferably of the same nature, for example, the main pump and each booster pump are centrifugal fans. Alternatively, the main pump and a booster pump can be of different nature. Incidentally, a filtering apparatus is arranged upstream of main pump, with respect to the airflow and a booster pump can have specific seals and an impeller adapted to avoid clogging. A fluid, i.e. air eventually loaded with absorbent material, sucked in and expelled by a pump will gain in velocity and in pressure. Given that the booster pumps and the main pump are fluidically connected, with respect of the airflow, the main pump does not need to function at higher power to ensure a good vacuum throughout the manufacturing line and apparatus 120 since the booster pump(s) can ensure some of the vacuum. Such vacuum devices, or pumps are well adapted for this process and can ensure the proper vacuum forces described hereabove.

According to an embodiment, an exhaust hole can be arranged on the surface of the first conveying device 128 so that an air-blowing device can blow out air to push said portion of absorbent insert 4 upwards towards the static element 136.

According to an embodiment, the two webs of front and back panels 2,3 can be conveyed at same speed or at different speed. If the two webs of front and back panels 2,3 are conveyed at same speed, front and back panels 2,3 will have the same lengths with respect to the transversal direction or machine direction (MD) and the side seams will be arranged at the sides of the wearer. If the two webs of front and back panels 2,3 are conveyed at different speed, e.g. the web of back panel 3 is conveyed faster than the web of front panel 2 then the back panel 3 will be longer than the front panel 2 and the side seams will be more toward the front at the sides of the wearer.

According to an embodiment, the folding unit 134 comprises two static elements 136, e.g. two folding blades 136 or two folding rails 136, one arranged at each transversal edge of the absorbent insert 4, meaning one arranged at each end of the first conveying device 128 with respect to the cross-direction CD. The two static elements 136 are substantially identical in dimensions and shape and are preferably arranged in facing relationship or in mirror relationship, meaning at the same area with respect to the machine direction MD. Such arrangement enables to balance the force exerted onto the absorbent insert 4 and optionally to remove the pressing blade(s) 40. The rest of the process is arranged accordingly, for example an elastic thread(s) 12 is arranged between the absorbent insert 4 and the front panel which is folded onto the elastic thread(s) 12, the first adhesive dispensing device 102 dispenses the same amount of adhesive between the front and back panels 2,3 and the second adhesive dispensing device 104 dispenses the same amount of adhesive between both transversal edges of the absorbent insert 4. Such arrangement also enables to have a transversal waist barrier 5 in the front and in the back.

According to an embodiment the elastic material 11 sandwiched between the inner nonwoven layer 9 and the outer nonwoven layer 10 and the elastics strands 12 are different. The elastics 11,12 can differ in cross-sectional shapes, e.g. round or flat, in dtex, in composition and/or in color. Alternatively, said elastic material 11 and elastic strands 12 can comprise, preferably consist of, the same material.

The present disclosure is not limited to these embodiments, the method and process can comprise the placing of leg cuffs and leg elastics, wetness indicators, etc.

## Claims

1. A method for the manufacture of an absorbent article (1) comprising the steps of:
• providing a front panel (2);
• providing a back panel (3);
• providing an absorbent insert (4) comprising front and back transversal edges and left and right longitudinal edges connecting the front and back transversal edges to form a perimeter thereof;
• joining said absorbent insert (4) to said front and/or back panels (2, 3) followed by folding a portion of said absorbent insert (4) proximal to the front and/or back transversal edges thereof onto itself, preferably in the shape of a substantially U-fold, to form transversal waist barrier(s) (5); and
• joining said front and back panels (2, 3) together along a pair of oppositely disposed side seams.

2. A method according to claim 1 further comprising the steps of folding an outer nonwoven layer (10) of the front and/or back panels (2, 3) over the folded absorbent insert (4) and joining said folded outer nonwoven layer (10) directly or indirectly to the folded absorbent insert (4).

3. A method according to claim 2 further comprising the step of arranging an elastic material (12) over the folded portion of the folded absorbent insert (4) prior to step of folding an outer nonwoven layer (10) of the front and/or back panels (2, 3) over the folded absorbent insert (4).

4. A method according to any of the claims 1 to 3 further comprising the step of intermittently applying adhesive onto said portion of the absorbent insert (4) proximal to the front and/or back transversal edges.

5. A method according to any of the claims 1 to 3 further comprising the step of applying adhesive onto said portion of the absorbent insert (4) proximal to the front and/or back transversal edges in an adhesive pattern (71), said adhesive pattern (71) comprising at least two areas (70) of adhesive arranged at the transversal extremities of the front and/or back transversal edges, a further area (72) of adhesive extending transversally between said two areas (70) of adhesive, said further area (72) being at a longitudinal distance from the transversal edge, and an adhesive-free area (73) arranged between the transversal edge of the absorbent insert (4) and said further area (72).

6. A method according to any of the claims 1 to 5 further comprising the step of compressing the folded portion of the folded absorbent insert (4), preferably at least the transversal waist barrier(s) (5), once formed prior to the steps of folding an outer nonwoven layer (10) of the front and/or back panels (2, 3) over the folded absorbent insert (4) and joining said folded outer nonwoven layer (10) directly or indirectly to the folded absorbent insert (4).

7. A method according to claim 3 to 6 further comprising the step of strand coating the elastic material (12) prior to the step of arranging said elastic material (12) over the folded portion of the folded absorbent insert (4).

8. A method according to any of the claims 2 to 7 further comprising the step of compressing the folded portion of the folded absorbent insert (4) once the outer nonwoven layer (10) has been joined directly or indirectly to the folded absorbent insert (4).

9. A method according to any of the claims 1 to 8 further comprising the step of mechanically bonding the folded transversal edge of the absorbent insert (4) once the transversal waist barrier(s) (5) has been formed.

10. Apparatus (120) for the manufacture of an absorbent article (1) according to a method according to any of the claims 1 to 9, wherein the apparatus (120) comprises:
• a conveying device (128), preferably a conveyor belt (128), for conveying said front and/or back panels (2,3) and for joining said absorbent insert (4) to said front and/or back panels (2, 3);
• a folding unit (34) for folding a portion of said absorbent insert (4) proximal to the front and/or back transversal edges thereof onto itself, preferably in the shape of a substantially U-fold, to form transversal waist barrier(s) (5) said absorbent insert (4) being joined to said front and/or back panel (2,3);
• a bonding unit for joining the joining said front and back panels (2, 3) together along a pair of oppositely disposed side seams.

11. Apparatus according to claim 10, wherein the conveying device (128) extends in the vertical direction (Z), said apparatus (120) extending in a machine direction (MD), a cross-direction (CD) and a vertical direction (Z).

12. Apparatus according to claim 10 or 11, wherein the folding unit (134) comprises, preferably consists of, a static element (136) preferably in the form of a rail or a rod or a plate or a blade .

13. Apparatus according to any of the claims 10 to 12, wherein the apparatus (120) comprises an adhesive dispensing device (104) arranged upstream or downstream of the folding unit (134) with respect to the machine direction (MD), said device (32) being configured to apply adhesive intermittently.

14. Apparatus according to any of the claims 10 to 13, wherein a pressure roller (146) is arranged to press the folded transversal edge of the absorbent insert (4), said pressure roller (46) being arranged with respect to the machine direction (MD) downstream of the folding unit (134) and upstream of an elastic depositing unit or station, where a second elastic material (12) is deposited onto the backsheet (7) or the outer cover (7') of the absorbent insert (4) at the region that has been folded over.

## Patentansprüche

1. Verfahren zum Herstellen eines Absorptionsartikels (1), umfassend die Schritte:
• Bereitstellen einer Vorderseite (2);
• Bereitstellen einer Rückseite (3);
• Bereitstellen einer Absorptionseinlage (4), umfassend einen vorderen und einen hinteren Querrand und einen linken und einen rechten Längsrand, die den vorderen und den hinteren Querrand verbinden, um einen Umfang davon auszubilden;
• Verknüpfen der Absorptionseinlage (4) mit der Vorder- und/oder Rückseite (2, 3), gefolgt durch ein Falten eines Abschnitts der Absorptionseinlage (4) proximal zu dem vorderen und/oder dem hinteren Querrand davon auf sich selbst, vorzugsweise in der Form einer im Wesentlichen U-förmigen Falte, um (eine) Quertaillenbarriere(n) (5) auszubilden; und
• Verknüpfen der Vorder- und der Rückseite (2, 3) miteinander entlang eines Paars von gegenüberliegend angeordneten Seitennähten.

2. Verfahren nach Anspruch 1, ferner umfassend die Schritte des Faltens einer äußeren Vliesschicht (10) der Vorder- und/oder der Rückseite (2, 3) über die gefaltete Absorptionseinlage (4) und des direkten oder indirekten Verknüpfens der gefalteten äußeren Vliesschicht (10) mit der gefalteten Absorptionseinlage (4).

3. Verfahren nach Anspruch 2, ferner umfassend den Schritt eines Einrichtens eines elastischen Materials (12) über dem gefalteten Abschnitt der gefalteten Absorptionseinlage (4) vor dem Schritt des Faltens einer äußeren Vliesschicht (10) der Vorder- und/oder der Rückseite (2, 3) über die gefaltete Absorptionseinlage (4).

4. Verfahren nach einem der Ansprüche 1 bis 3, ferner umfassend den Schritt eines intermittierenden Auftragens von Klebstoff auf den Abschnitt der Absorptionseinlage (4) in der Nähe des vorderen und/oder des hinteren Querrands.

5. Verfahren nach einem der Ansprüche 1 bis 3, ferner umfassend den Schritt des Auftragens von Klebstoff auf den Abschnitt der Absorptionseinlage (4) in der Nähe des vorderen und/oder des hinteren Querrands in einem Klebstoffmuster (71), das Klebstoffmuster (71) umfassend mindestens zwei Klebstoffbereiche (70), die an den Querextremitäten des vorderen und/oder des hinteren Querrands eingerichtet sind, wobei sich ein weiterer Klebstoffbereich (72) zwischen den zwei Klebstoffbereichen (70) quer erstreckt, wobei der weitere Bereich (72) in einem Längsabstand von dem Querrand ist, und wobei ein Klebstoff freier Bereich (73) zwischen dem Querrand der Absorptionseinlage (4) und dem weiteren Bereich (72) eingerichtet ist.

6. Verfahren nach einem der Ansprüche 1 bis 5, ferner umfassend den Schritt eines Zusammendrückens des gefalteten Abschnitts der gefalteten Absorptionseinlage (4), vorzugsweise mindestens der Quertaillenbarriere(n) (5), sobald ausgebildet, vor den Schritten des Faltens einer äußeren Vliesschicht (10) der Vorder- und/oder der Rückseite (2, 3) über die gefaltete Absorptionseinlage (4) und des direkten oder indirekten Verknüpfens der gefalteten äußeren Vliesschicht (10) mit der gefalteten Absorptionseinlage (4).

7. Verfahren nach einem der Ansprüche 3 bis 6, ferner umfassend den Schritt eines Strangbeschichtens des elastischen Materials (12) vor dem Schritt des Einrichtens des elastischen Materials (12) über dem gefalteten Abschnitt der gefalteten Absorptionseinlage (4).

8. Verfahren nach einem der Ansprüche 2 bis 7, ferner umfassend den Schritt des Zusammendrückens des gefalteten Abschnitts der gefalteten Absorptionseinlage (4), sobald die äußere Vliesschicht (10) direkt oder indirekt mit der gefalteten Absorptionseinlage (4) verknüpft wurde.

9. Verfahren nach einem der Ansprüche 1 bis 8, ferner umfassend den Schritt eines mechanischen Bindens des gefalteten Querrands der Absorptionseinlage (4), sobald die Quertaillenbarriere(n) (5) ausgebildet worden ist/sind.

10. Einrichtung (120) für die Herstellung eines Absorptionsartikels (1) gemäß einem Verfahren nach einem der Ansprüche 1 bis 9, wobei die Einrichtung (120) umfasst:
• eine Fördervorrichtung (128), vorzugsweise ein Förderband (128), zum Fördern der Vorder- und/oder der Rückseite (2, 3) und zum Verknüpfen der Absorptionseinlage (4) mit der Vorder- und/oder der Rückseite (2, 3);
• eine Falteinheit (34) zum Falten eines Abschnitts der Absorptionseinlage (4) in der Nähe des vorderen und/oder des hinteren Querrands davon auf sich selbst, vorzugsweise in der Form einer im Wesentlichen U-förmigen Falte, um (eine) Quertaillenbarriere(n) (5) der Absorptionseinlage (4) auszubilden, die mit der Vorder- und/oder der Rückseite (2, 3) verknüpft wird;
• eine Bindeeinheit zum Verknüpfen der Vorder- und der Rückseite (2, 3) miteinander entlang eines Paars von gegenüberliegend angeordneten Seitennähten.

11. Einrichtung nach Anspruch 10, wobei sich die Fördervorrichtung (128) in vertikaler Richtung (Z) erstreckt, wobei sich die Einrichtung (120) in Maschinenrichtung (MD), Querrichtung (CD) und vertikaler Richtung (Z) erstreckt.

12. Einrichtung nach Anspruch 10 oder 11, wobei die Falteinheit (134) ein statisches Element (136) umfasst, vorzugsweise aus diesem besteht, vorzugsweise in der Form einer Schiene, einer Stange, einer Platte oder einer Klinge.

13. Einrichtung nach einem der Ansprüche 10 bis 12, wobei die Einrichtung (120) eine Klebstoffabgabevorrichtung (104) umfasst, die stromaufwärts oder stromabwärts von der Falteinheit (134) in Bezug auf die Maschinenrichtung (MD) eingerichtet ist, wobei die Vorrichtung (32) konfiguriert ist, um Klebstoff intermittierend aufzutragen.

14. Einrichtung nach einem der Ansprüche 10 bis 13, wobei eine Druckwalze (146) eingerichtet ist, um die gefaltete Querkante der Absorptionseinlage(4) anzudrücken, wobei die Druckwalze (46) in Bezug auf die Maschinenrichtung (MD) stromabwärts von der Falteinheit (134) und stromaufwärts von einer elastischen Ablageeinheit oder -station eingerichtet ist, wo ein zweites elastisches Material (12) auf die rückseitige Lage (7) oder die Außenhülle (7') der Absorptionseinlage (4) in der Region, die umgefaltet wurde, aufgebracht wird.

## Revendications

1. Procédé permettant la fabrication d'un article absorbant (1) comprenant les étapes consistant à :
• fournir un panneau avant (2) ;
• fournir un panneau arrière (3) ;
• fournir un insert absorbant (4) comprenant des bords transversaux avant et arrière et des bords longitudinaux gauche et droit reliant les bords transversaux avant et arrière pour en former le périmètre ;
• joindre ledit insert absorbant (4) auxdits panneaux avant et/ou arrière (2, 3), puis plier sur elle-même une partie dudit insert absorbant (4) à proximité de ses bords transversaux avant et/ou arrière, de préférence sous la forme d'un pli sensiblement en U, pour former une ou plusieurs barrières transversales à la taille (5) ; et
• joindre lesdits panneaux avant et arrière (2, 3) ensemble le long d'une paire de coutures latérales disposées de manière opposée.

2. Procédé selon la revendication 1, comprenant en outre les étapes consistant à plier une couche extérieure non tissée (10) des panneaux avant et/ou arrière (2, 3) sur l'insert absorbant plié (4) et à joindre ladite couche extérieure non tissée pliée (10) directement ou indirectement à l'insert absorbant plié (4).

3. Procédé selon la revendication 2, comprenant en outre l'étape consistant à agencer un matériau élastique (12) sur la partie pliée de l'insert absorbant plié (4) avant l'étape consistant à plier une couche extérieure non tissée (10) des panneaux avant et/ou arrière (2, 3) sur l'insert absorbant plié (4).

4. Procédé selon l'une quelconque des revendications 1 à 3, comprenant en outre l'étape consistant à appliquer de manière intermittente un adhésif sur ladite partie de l'insert absorbant (4) à proximité des bords transversaux avant et/ou arrière.

5. Procédé selon l'une quelconque des revendications 1 à 3, comprenant en outre l'étape consistant à appliquer de l'adhésif sur ladite partie de l'insert absorbant (4) à proximité des bords transversaux avant et/ou arrière dans un motif adhésif (71), ledit motif adhésif (71) comprenant au moins deux zones (70) d'adhésif disposées au niveau des extrémités transversales des bords transversaux avant et/ou arrière, une zone supplémentaire (72) d'adhésif s'étendant transversalement entre lesdites deux zones (70) d'adhésif, ladite zone supplémentaire (72) étant à une distance longitudinale du bord transversal, et une zone sans adhésif (73) étant prévue entre le bord transversal de l'insert absorbant (4) et ladite zone supplémentaire (72).

6. Procédé selon l'une quelconque des revendications 1 à 5, comprenant en outre l'étape consistant à comprimer la partie pliée de l'insert absorbant plié (4), de préférence au moins la ou les barrières transversales à la taille (5), une fois formées avant les étapes consistant à plier une couche extérieure non tissée (10) des panneaux avant et/ou arrière (2, 3) sur l'insert absorbant plié (4) et à joindre ladite couche extérieure non tissée pliée (10) directement ou indirectement à l'insert absorbant plié (4).

7. Procédé selon les revendications 3 à 6, comprenant en outre l'étape consistant à enrober le matériau élastique (12) avant l'étape consistant à agencer ledit matériau élastique (12) sur la partie pliée de l'insert absorbant plié (4).

8. Procédé selon l'une quelconque des revendications 2 à 7, comprenant en outre l'étape consistant à comprimer la partie pliée de l'insert absorbant plié (4) une fois que la couche extérieure non tissée (10) a été jointe directement ou indirectement à l'insert absorbant plié (4).

9. Procédé selon l'une quelconque des revendications 1 à 8, comprenant en outre l'étape consistant à coller de manière mécanique le bord transversal plié de l'insert absorbant (4) une fois que la ou les barrières transversales à la taille (5) ont été formées.

10. Appareil (120) permettant la fabrication d'un article absorbant (1) selon un procédé selon l'une quelconque des revendications 1 à 9, dans lequel l'appareil (120) comprend :
• un dispositif de transport (128), de préférence une bande transporteuse (128), pour transporter lesdits panneaux avant et/ou arrière (2, 3) et pour joindre ledit insert absorbant (4) auxdits panneaux avant et/ou arrière (2, 3) ;
• une unité de pliage (34) pour plier sur elle-même une partie dudit insert absorbant (4) à proximité de ses bords transversaux avant et/ou arrière, de préférence sous la forme d'un pli sensiblement en U, pour former une ou plusieurs barrières transversales à la taille (5), ledit insert absorbant (4) étant joint aux panneaux avant et/ou arrière (2, 3) ;
• une unité de collage pour joindre lesdits panneaux avant et arrière joints (2, 3) le long d'une paire de coutures latérales disposées de manière opposée.

11. Appareil selon la revendication 10, dans lequel le dispositif de transport (128) s'étend dans la direction verticale (Z), ledit appareil (120) s'étendant dans un sens machine (MD), un sens transversal (CD) et un sens vertical (Z).

12. Appareil selon la revendication 10 ou 11, dans lequel l'unité de pliage (134) comprend, de préférence consiste en un élément statique (136), de préférence sous la forme d'un rail, d'une tige, d'une plaque ou d'une lame.

13. Appareil selon l'une quelconque des revendications 10 à 12, dans lequel l'appareil (120) comprend un dispositif de distribution d'adhésif (104) disposé en amont ou en aval de l'unité de pliage (134) par rapport au sens machine (MD), ledit dispositif (32) étant conçu pour appliquer de l'adhésif par intermittence.

14. Appareil selon l'une quelconque des revendications 10 à 13, dans lequel un rouleau presseur (146) est disposé pour presser le bord transversal plié de l'insert absorbant (4), ledit rouleau presseur (46) étant disposé par rapport au sens machine (MD) en aval de l'unité de pliage (134) et en amont d'une unité ou station de dépôt d'élastique, où un second matériau élastique (12) est déposé sur la feuille arrière (7) ou le revêtement extérieur (7') de l'insert absorbant (4) au niveau de la zone qui a été repliée.
